(19) Europäisches Patentamt
European Patent Office
Office européen des brevets

(11) Veröffentlichungsnummer: **0 430 078 A2**

(12) **EUROPÄISCHE PATENTANMELDUNG**

(21) Anmeldenummer: **90122333.9**

(22) Anmeldetag: **22.11.90**

(51) Int. Cl.5: **C07J 51/00**, C07J 9/00, A61K 31/575

(30) Priorität: **22.11.89 CH 4183/89**

(43) Veröffentlichungstag der Anmeldung: **05.06.91 Patentblatt 91/23**

(84) Benannte Vertragsstaaten: **AT BE CH DE DK ES FR GB GR IT LI LU NL SE Patentblatt**

(71) Anmelder: **F. HOFFMANN-LA ROCHE AG**
**Postfach 3255**
**CH-4002 Basel(CH)**

(72) Erfinder: **Cassal, Jean-Marie, Dr.**
**4 rue du Markstein**
**F-68100 Mulhouse(FR)**
Erfinder: **Gains, Nigel, Dr.**
**Bläsiring 88**
**CH-4057 Basel(CH)**
Erfinder: **Gutknecht, Eva-Maria, Dr.**
**Im Feldele 23**
**W-7845 Buggingen-Seefelden(DE)**
Erfinder: **Hirth, Georges**
**7 rue de l'Ancre**
**F-68330 Huningue(FR)**
Erfinder: **Lengsfeld, Hans, Dr.**
**Mittlere Strasse 43**
**CH-4056 Basel(CH)**

(74) Vertreter: **Lederer, Franz, Dr. et al**
**Lederer, Keller & Riederer Patentanwälte**
**Lucile-Grahn-Strasse 22**
**W-8000 München 80(DE)**

(54) **Steroide.**

(57) Steroide der Formel

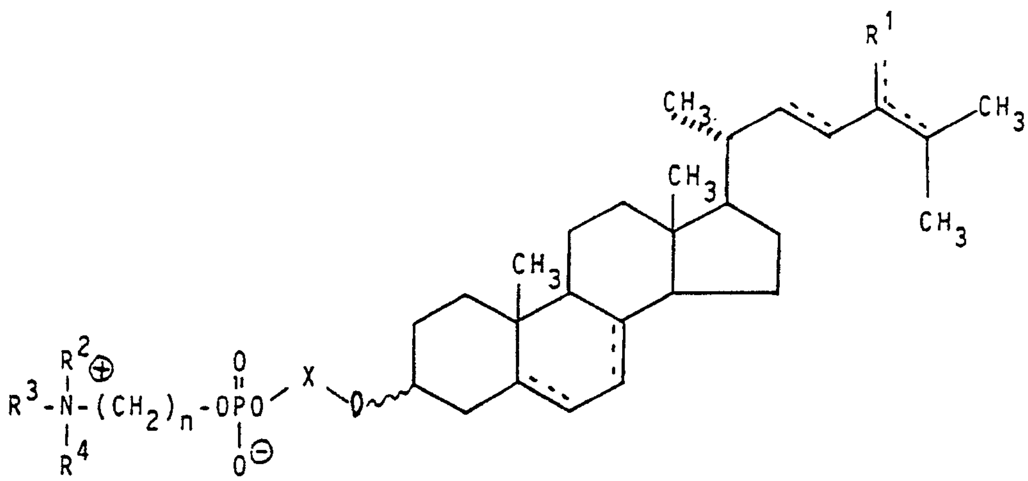

I

worin R1 bis R4, X und n die in der Beschreibung angegebene Bedeutung haben, besitzen die intestinale Resorption von Cholesterin und das Plasmacholesterin senkende Wirksamkeit. Sie werden hergestellt ausgehend von entsprechenden Steroiden mit einem Alkoholrest der Formel -O-X-OH in 3-Stellung.

EP 0 430 078 A2

## STEROIDE

Die Erfindung betrifft neue Steroide der Formel

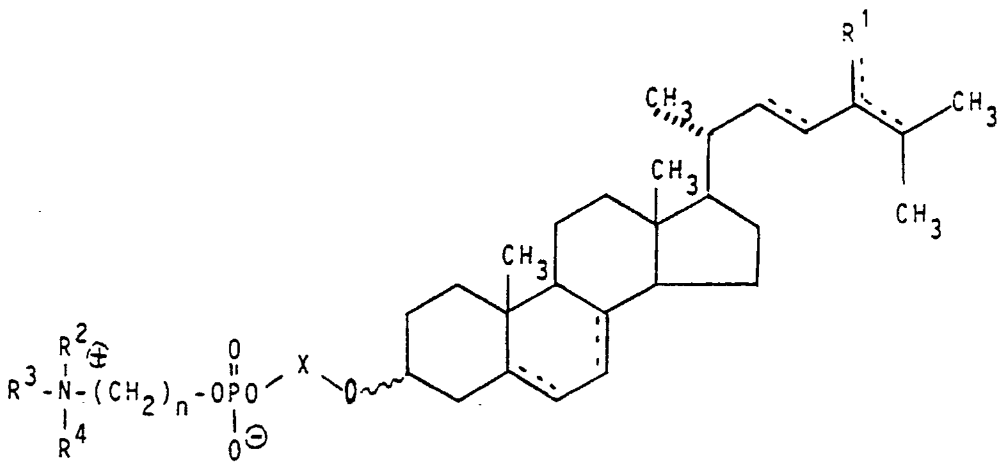

I

worin

$R^1$ Wasserstoff, nieder-Alkyl oder nieder-Alkyliden,

$R^2$, $R^3$ und $R^4$ Wasserstoff oder nieder-Alkyl sind oder zusammen mit dem N-Atom einen 5- oder 6-gliedrigen, gegebenenfalls niederalkylierten, aromatischen oder gesättigten heterocyclischen Rest bilden,

n die Zahl 2, 3 oder 4,

X eine Gruppe der Formel

$-(CH_2)_p-C(Q,Q')-(Z)_{1\ \text{oder}\ 0}-$,

$-CH_2CH(Y)CH_2-(Z)_{1\ \text{oder}\ 0}-$,

$-CH_2CH(CH_2Y)-(Z)_{1\ \text{oder}\ 0}-$ oder

$-(CH_2CH_2O)_q-CH_2CH_2-(Z)_{1\ \text{oder}\ 0}-$,

q die Zahl 1 oder 2,

Z eine Gruppe der Formel $-C(O)-$, $-OC(O)-$, $-OC(O)CH_2-$, $-OCH_2C(O)-$ oder $-N(T)C(O)-$,

Q, Q' und T Wasserstoff oder nieder-Alkyl,

p eine ganze Zahl zwischen 1 und 9 ist und, falls Z Carbonyl ist, auch 0 sein kann,

Y Hydroxy, nieder-Alkoxy, nieder-Alkanoyloxy, Carbamoyloxy oder Mono- oder Di-niederalkyl-carbamoyloxy ist, die gestrichelten C-C-Bindungen in 5(6)-, 7(8)-, 22(23)-, 24(25)- und 24(28)-Stellung fakultativ sind, wobei die Seitenkette entweder gesättigt oder einfach ungesättigt ist,

und physiologisch verträgliche Salze davon.

Die an den C-Atomen in Stellung 3 und 5 des Steroidanteils der Verbindungen der Formel I gebundenen Wasserstoffatome können unabhängig voneinander die α- oder β-Konfiguration haben.

Die Erfindung betrifft weiterhin ein Verfahren zur Herstellung dieser Steroide, Arzneimittel auf der Basis letzterer und diese Steroide als pharmazeutische Wirkstoffe, sowie die Verwendung dieser Steroide bei der Herstellung von die intestinale Resorption von Cholesterin hemmenden und das Plasmacholesterin senkenden Medikamenten.

Der Ausdruck "nieder" bedeutet, dass die derart bezeichneten Reste bis zu 4 Kohlenstoffatome enthalten und geradkettig oder verzweigt sein können. Beispiele von nieder-Alkylresten sind Methyl, Aethyl, n-Propyl, Isopropyl, n-Butyl und t-Butyl. Beispiele von nieder-Alkylidenresten sind Methylen und Aethyliden. Beispiele von nieder-Alkanoyloxyresten sind Acetoxy und Propionyloxy.

Pharmazeutisch annehmbare Salze der Steroide der Formel I sind anorganische Salze, wie Hydrochloride und Sulfate; organische Salze, wie Trifluoracetate, Mesylate und Tosylate; und Metallsalze, wie Natriumsalze. Die Verbindungen der Formel I können in Form eines Zwitterions vorliegen und diejenigen, in denen mindestens einer der Reste $R^2$, $R^3$ und $R^4$ Wasserstoff ist, können in Form eines Hydrogenphosphats vorliegen.

Unter den Steroiden der Formel I sind diejenigen bevorzugt, worin $R^1$ Wasserstoff oder nieder-Alkyl, insbesondere Aethyl ist, ferner diejenigen, worin $R^2$ Wasserstoff oder nieder-Alkyl und $R^3$ und $R^4$ nieder-

Alkyl, insbesondere Methyl sind, oder worin $R^2$, $R^3$ und $R^4$ zusammen mit dem N-Atom eine Pyridinium- oder 1-Methylpyrrolidiniumgruppe bilden.

Bevorzugt sind ferner die Steroide der Formel I, worin n die Zahl 2 ist, sowie diejenigen mit gesättigtem oder 5(6)-ungesättigtem oder 5(6)- und 22(23)-ungesättigtem Steroidanteil.

Weitere bevorzugte Steroide der Formel I sind diejenigen, worin X die Gruppe $-(CH_2)_2-$, $-(CH_2)_2O(CH_2)_2-$, $-(CH_2)_2O(CH_2)_2O(CH_2)_2-$, $-CH_2CHOHCH_2-$, $-CH_2CH(OCOCH_3)CH_2-$, $-CH_2CH(OCONHCH_3)CH_2N[CH(CH_3)_2]CO-$, $-(CH_2)_2NHCO-$, $-(CH_2)_3NHCO-$, $-CH_2CHOHCH_2OCO-$, $-CH(CH_3)CO-$ oder insbesondere $-(CH_2)_2OCO-$, $-(CH_2)_3OCO-$, $-(CH_2)_4OCO-$, $-(CH_2)_8OCO-$, $-(CH_2)CO-$, $-(CH_2)_2CO-$, $-(CH_2)_3CO-$, $-(CH_2)_5CO-$, $-CH_2CH(OCOCH_3)CH_2OCO-$, $-CH_2C(CH_3)_2CO-$ oder $-CH_2CH(CH_2OH)NHCO-$ darstellt.

Beispiele von bevorzugten Verbindungen der Formel I sind die folgenden:
O-[[2-[[(Cholest-5-en-3β-yloxy)carbonyl]oxy]äthoxy]hydroxyphosphinyl]cholinhydroxid-inneres Salz,
O-[Hydroxy-[3-[(3β-stigmastanyloxy)carbonyl]propoxy]phosphinyl]cholinhydroxid-inneres Salz,
O-[[2-(Cholest-5-en-3β-yloxy)äthoxy]hydroxyphosphinyl]cholinhydroxid-inneres Salz und
O-[Hydroxy-[2-[1-(5α-stigmastan-3β-yloxy)formamido]äthoxy]phosphinyl]cholinhydroxid-inneres Salz.

Weitere Beispiele von Verbindungen der Formel I sind die folgenden:
O-[[2-[[(5α-Stigmastan-3β-yloxy)carbonyl]oxy]äthoxy]hydroxyphosphinyl]cholinhydroxid-inneres Salz,
O-[Hydroxy-[2-[[[E)-stigmasta-5,22-dien-3β-yloxy]carbonyl]äthoxy]phosphinyl]cholinhydroxid-inneres Salz,
O-[[3-[[(Cholest-5-en-3β-yloxy)carbonyl]oxy]propoxy]hydroxyphosphinyl]cholinhydroxid-inneres Salz,
O-[[4-[[(Cholest-5-en-3β-yloxy)carbonyl]oxy]butoxy]hydroxyphosphinyl]cholinhydroxid-inneres Salz,
O-[[[8-[[(Cholest-5-en-3β-yloxy)carbonyl]oxy]octyl]oxy]hydroxyphosphinyl]cholinhydroxid-inneres Salz,
O-[[[(Cholest-5-en-3β-yloxy)carbonyl]methoxy]hydroxyphosphinyl]cholinhydroxid-inneres Salz,
O-[[2-[(Cholest-5-en-3β-yloxy)carbonyl]äthoxy]hydroxyphosphinyl]cholinhydroxid-inneres Salz,
O-[Hydroxy-[2-[(3β-stigmastanyloxy)carbonyl]äthoxy]phosphinyl]cholinhydroxid-inneres Salz,
O-[Hydroxy-[2-[(stigmasta-5,22-dien-3β-yloxy)carbonyl]äthoxy]phosphinyl]cholinhydroxid-inneres Salz,
O-[[3-[(Cholest-5-en-3β-yloxy)carbonyl]propoxy]hydroxyphosphinyl]cholinhydroxid-inneres Salz,
O-[Hydroxy-[3-[(E)-stigmasta-5,22-dien-3β-yloxy)carbonyl]propoxy]phosphinyl]cholinhydroxid-inneres Salz,
O-[Hydroxy-[[5-[(5α-stigmastan-3β-yloxy)carbonyl]pentyl]oxy]phosphinyl]cholinhydroxid-inneres Salz,
O-[Hydroxy-[[5-[((E)-stigmasta-5,22-dien-3β-yloxy)carbonyl]pentyl]oxy]phosphinyl]cholinhydroxid-inneres Salz,
O-[Hydroxy-[[[(3β-stigmastanyl)oxy]carbonyl]methoxy]phosphinyl]cholinhydroxid-inneres Salz,
O-[Hydroxy-[[[(E)-stigmasta-5,22-dien-3β-yloxy]carbonyl]methoxy]phosphinyl]cholinhydroxid-inneres Salz,
O-[Hydroxyl-[2-(stigmasta-5,22-dien-3β-yloxy)äthoxy]phosphinyl]cholinhydroxid-inneres Salz,
O-[[2-[2-(Cholest-5-en-3β-yloxy)äthoxy]äthoxy]hydroxyphosphinyl]cholinhydroxid-inneres Salz,
O-[Hydroxy-[2-[2-[(E)-stigmasta-5,22-dien-3β-yloxy]äthoxy]äthoxy]phosphinyl]cholinhydroxid-inneres Salz,
O-[[2-[2-[2-(Cholest-5-en-3β-yloxy)äthoxy]äthoxy]äthoxy]hydroxyphosphinyl]cholinhydroxid-inneres Salz,
O-[[2-[(5α-Cholestan-3β-yloxy)carbonyloxy]äthoxy]hydroxyphosphinyl)cholinhydroxid-inneres Salz,
O-[Hydroxy-[2-[2-(3β-stigmastanyloxy)äthoxy]äthoxy]phosphinyl]cholinhydroxid-inneres Salz,
O-[Hydroxy-[2-(3β-stigmastanyloxy)äthoxy]phosphinyl]cholinhydroxid-inneres Salz,
1-[2-[[Hydroxy-[2-[1-(5α-stigmastan-3β-yloxy)formamido]äthoxy]phosphinyl]oxy]äthyl]pyridiniumhydroxid-inneres Salz,
O-[Hydroxy-[2-[1-[(E)-stigmasta-5,22-dien-3β-yloxy]formamido]äthoxy]phosphinyl]cholinhydroxid-inneres Salz,
O-[Hydroxy-[3-[1-[(E)-stigmasta-5,22-dien-3β-yloxy]formamido]propoxy]phosphinyl]cholinhydroxid-inneres Salz,
O-[Hydroxy-[(RS)-3-[N-isopropyl-1-[(E)-stigmasta-5,22-dien-3β-yl)oxyformamido]-2-[(methylcarbamoyl)oxy]-propoxy]phosphinyl]cholinhydroxid-inneres Salz,
O-[[[2-[1-Cholest-5-en-3β-yloxy]formamido[äthoxy]hydroxyphosphinyl]cholinhydroxid-inneres Salz,
O-[[2-[1-(5β-Cholestan-3α-yloxy)formamido]äthoxy]hydroxyphosphinyl]cholinhydroxid-inneres Salz,
1-[2-[[Hydroxy-[3-[1-[(E)-stigmasta-5,22-dien-3β-yloxy]formamido]propoxy]phosphinyl]oxy]äthyl]-pyridiniumhydroxid-inneres Salz,
O-[Hydroxy-[2-[[[(E)-stigmasta-5,22-dien-3β-yloxy]carbonyl]oxy]äthoxy]phosphinyl]cholinhydroxid-inneres Salz,
1-[2-[[[2-[1-[Cholest-5-en-3β-yloxy]formamido]äthoxy]hydroxyphosphinyl]oxyjäthyl]-1-methylpyrrolidiniumhydroxid-inneres Salz,
1-O-(3α,β-Stigmastanyl)-3-O-(RS)-glyceryl-phosphorylcholin,
O-[[(RS)-2-Acetoxy-3-[5α-stigmastan-3α,β-yloxy]propoxy]hydroxyphosphonyl]cholinhydroxid-inneres Salz,
O-[[(RS)-3-[(cholest-5-en-3β-yloxy)carbonyloxy]-2-hydroxypropoxy]hydroxyphosphinyl]cholinhydroxid-inneres Salz,

O-[Hydroxy-(RS)-2-hydroxy-3-[[5α-stigmastan-3β-yloxy)carbonyloxy]propoxy]phosphinyl]cholinhydroxid-inneres Salz

O-[Hydroxy-[(RS)-2-hydroxy-3-[[(E)stigmasta-5,22-dien-3β-yloxy]carbonyloxy]propoxy]phosphinyl]-cholinhydroxid-inneres Salz,

O-[Hydroxy-[2-[[(stigmast-5-en-3β-yloxy)carbonyl]oxy]äthoxy]phosphinyl]cholinhydroxid-inneres Salz,

O-[Hydroxy-[3-[(stigmast-5-en-3β-yloxy)carbonyl]propoxy]phosphinyl]cholinhydroxid-inneres Salz,

O-[Hydroxy-[2-(stigmast-5-en-3β-yloxy)äthoxy]phosphinyl]cholinhydroxid-inneres Salz,

Cholest-5-en3β-yl-2-[[[2-(dimethylamino)äthoxy]hydroxyphosphinyl]oxy]äthylcarbonat,

O-[Hydroxy-[2-[1-(stigmast-5-en-3β-yloxy)formamido]äthoxy]phosphinyl]cholinhydroxid-inneres Salz,

O-[[(RS)-2-Acetoxy-3-[[(cholest-5-en-3β-yloxy)carbonyl]oxy]propoxy]hydroxyphosphinyl]cholinhydroxid-inneres Salz,

O-[[2-[cholest-5-en-3β-yloxy)carbonyl]-2-methylpropoxy]hydroxyphosphinyl]-cholinhydroxid-inneres Salz und

O-[[(RS)-2-[1-(Cholest-5-en-3β-yloxy)formamido)-3-hydroxypropoxy]hydroxyphosphinyl]cholinhydroxid-inneres Salz.

Die Steroide der Formel I und die Salze davon kann man dadurch herstellen, dass man

a) einen Alkohol der Formel

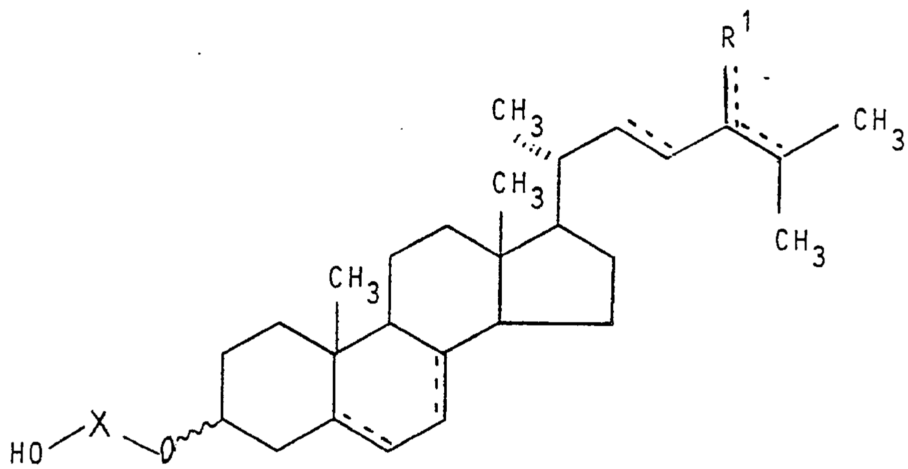

II

unter intermediärem Schutz eines in der Gruppe X vorhandenen Hydroxyrestes Y, mit einem die Gruppe der Formel

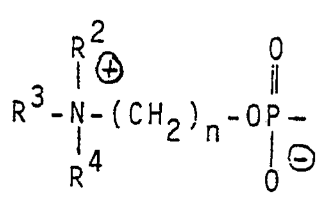

(G)

einführenden Mittel behandelt, und

b) gewünschtenfalls ein ungesättigtes Steroid der Formel I zum gesättigten Steroid hydriert,

c) gewünschtenfalls einen in der Gruppe X eines Steroids der Formel I enthaltenen reaktionsfähigen Rest funktionell abwandelt,

d) gewünschtenfalls ein Steroid der Formel I in ein Salz überführt.

Diese Reaktionen kann man in an sich bekannter Weise durchführen.

So kann ein Carbonat der Formel II, worin X eine Gruppe der Formel $-(CH_2)_p-OCO-$ ist, in einem Lösungsmittel, wie Methylenchlorid oder Chloroform, in Gegenwart von einer Base, wie Chinolin oder Pyridin, zunächst mit einem Halogenid der Formel $PO(Hal)_3$, z.B. Phosphoroxychlorid, bei Raumtemperatur umgesetzt und die erhaltene Verbindung mit einem Salz der Formel

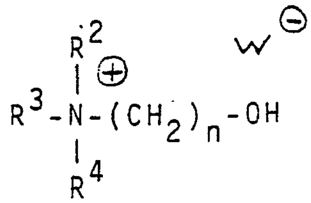

III

worin W nieder-Alkylsulfonyloxy oder Arylsulfonyloxy ist, z.B. mit dem Cholintosylat, in Gegenwart einer Base, wie Pyridin, in einem Lösungsmittel, wie Methylenchlorid, bei Raumtemperatur versetzt werden.

Ein Carbamat der Formel II, worin X eine Gruppe der Formel $-(CH_2)_pN(T)C(O)-$ ist, kann man zunächst mit einem Halogenid der Formel

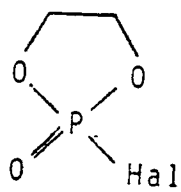

IV

z.B. mit 2-Chlor-2-oxo-1,3,2-dioxaphospholan, in einem Lösungsmittel wie Tetrahydrofuran (THF) unter Kühlung auf Temperaturen bis 0° C in Gegenwart einer Base, wie Triäthylamin, Umsetzen und das erhaltene Phosphat in dem gleichen Lösungsmittel unter Erhitzen, z.B. auf 50 bis 100° C, mit einem Amin der Formel $N(R^2,R^3,R^4)$ umsetzen.

Ein Ester der Formel II, worin X eine Gruppe der Formel $-CH(CH_3)CO-$ ist, kann man zunächst mit einem Halogenid, wie $\beta$-Bromäthylphosphorsäuredichlorid (Pharm. Acta Helv. 33, 1958, 349), in einem Lösungsmittel, wie Methylenchlorid in Gegenwart von einer Base, wie Triäthylamin umsetzen und die erhaltene Verbindung dann in einem Lösungsmittel, wie Toluol, mit einem Amin der Formel $N(R^2,R^3,R^4)$ versetzen.

Zum Schutz eines in der Gruppe X einer Verbindung der Formel II vorhandenen Hydroxyrestes Y kann man, wie im nachfolgenden Beispiel 11 beschrieben, eine Benzylgruppe verwenden. So kann man ein Glycerinderivat der Formel II, worin X eine Gruppe der Formel $-CH_2CH(OH)CH_2-$ ist, mit Tritylchlorid in Pyridin zum entsprechenden Tritylläther umsetzen. Dieser Aether lässt sich mittels Natriumhydrid in THF und Benzylchlorid in Dimethylformamid (DMF) bei einer Temperatur bis zu 100° C in den entsprechenden Trityl-benzyl-diäther überführen, worin X für $-CH_2CH(OCH_2C_6H_5)CH_2-$steht. Nach Abspaltung der Tritylgruppe aus dem Diäther, z.B. mittels Salzsäure in Dioxan unter Erhitzen, wird der erhaltene Benzyläther wie weiter oben beschrieben mit einem die Gruppe

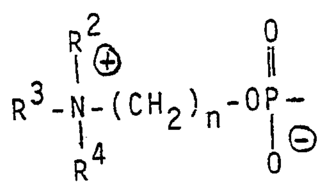

(G)

einführenden Mittel behandelt. Durch Abspaltung der Benzylgruppe, z.B. durch Hydrierung in Gegenwart von Palladium- Kohle (Pd/C) in Methanol und THF oder (zur Vermeidung der gleichzeitigen Hydrierung von im Steroidanteil des Aethers vorhandenen Doppelbindungen) in Gegenwart von Palladiumoxyd (PdO) in Essigsäure, erhält man das der Ausgangsverbindung der Formel II entsprechende Glycerinderivat der Formel I, worin X $-CH_2CH(OH)CH_2-$ ist.

Ein benzyl-geschützter Steroidalkohol der Formel II, worin X für $-CH_2CH(OCH_2C_6H_5)CH_2-$ steht, lässt sich auch durch Umsetzung des entsprechenden Steroid-3-chloroformiats mit O-Benzylglycerin in Methylenchlorid herstellen.

Zum Schutz eines in der Gruppe X einer Verbindung der Formel II vorhandenen Hydroxyrestes Y kann man ferner, wie im nachfolgenden Beispiel 22 beschrieben, eine Phenoxycarbonylgruppe verwenden. So kann man eine Verbindung der Formel II, worin X eine Gruppe $-CH_2CH(CH_2OH)NH-CO-$ ist, in Methylenchlorid, THF und Pyridin mit Phenylchloroformat umsetzen. Die erhaltene eine Phenoxycarbonyloxygruppe enthaltende Verbindung wird dann z.B. in Toluol und Triäthylamin mit einem die Gruppe der obigen Formel G einführenden Mittel, z.B. mit 2-Chlor-2-oxo-1,3,2-dioxaphospholan und dann mit Trimethylamin in Toluol und Acetonitril umgesetzt. Durch Abspaltung der Phenoxycarbonylgruppe, z.B. mittels wässrigem Natriumhydroxid, erhält man die Verbindung der Formel I, in der X die Gruppe $-CH_2CH(CH_2OH)NHCO-$ ist.

Die Hydrierung eines ungesättigten Steroids der Formel I kann man in Gegenwart von Pd/C in einem Lösungsmittel, wie Methanol, bei einer Temperatur bis zu 100 °C durchführen.

Als funktionelle Abwandlung eines in der Gruppe X eines Steroids der Formel I enthaltenen reaktionsfähigen Restes, kann die Alkanoylierung einer Hydroxygruppe Y genannt werden. So kann man ein Glycerinderivat der Formel I, worin X die Gruppe $-CH_2CH(OH)CH_2-$ ist, in einem Lösungsmittel, wie Chloroform, in Gegenwart von einer Base, wie Pyridin, und einem Katalysator, wie Dimethylaminopyridin (DMAP), mit dem der einzuführenden Alkanoylgruppe entsprechenden Carbonsäureanhydrid bei Raumtemperatur umsetzen.

Die Ausgangsalkohole der Formel II, worin X eine Gruppe der Formel $-(CH_2)_p-C(Q,Q')-Z'$, $-CH_2CH(Y)-CH_2-Z'-$, $-CH_2CH(CH_2Y)-Z'-$ oder $-(CH_2CH_2O)_q-CH_2CH_2-Z'-$, Z' eine Gruppe der Formel -OC(O)-, $-OC(O)-CH_2-$, $-OCH_2C(O)-$ oder -N(T)C(O)- ist und $R^1$, Q, Q', p, q, Y und T die weiter oben angegebene Bedeutung haben, sind neu und als solche Gegenstand der Erfindung.

Die Alkohole der Formel II kann man in an sich bekannter Weise herstellen, z.B. wie beschrieben in den nachfolgenden Beispielen A bis M.

So kann man einen Alkoholäther der Formel II, worin X z.B. eine Gruppe $-(CH_2)_p-$ ist, durch Umsetzung des entsprechenden Steroid-3-tosylats mit dem Glykol $HO-(CH_2)_p-OH$ in Dioxan bei etwa 120 °C herstellen.

Einen Alkoholester der Formel II, worin X z.B. eine Gruppe $-(CH_2)_p-CO-$ ist, kann man herstellen durch Umsetzung des entsprechenden Steroid-3-ols in Methylenchlorid mit dem entsprechenden Halogenid der Formel $Hal-(CH_2)_p-COCl$ bei etwa 5 °C in Gegenwart von Pyridin und Ueberführung des erhaltenen Halogenids in den erwünschten Alkohol der Formel II mittels Kaliumtrifluoracetat in Methylenchlorid, DMF und Wasser bei etwa 80 °C.

Ein Alkoholester der Formel II, worin X z.B. eine Gruppe $-CH(CH_3)CO-$ ist, lässt sich herstellen durch Umsetzung des entsprechenden Steroid-3-ols mit Milchsäure in Toluol, in Gegenwart katalytischer Mengen von p-Toluolsulfonsäure.

Ein Alkoholcarbonat der Formel II, worin X z.B. eine Gruppe $-(CH_2)_p-OC(O)-$ ist, kann man herstellen durch Umsetzung des entsprechenden Steroid-3-ols in Methylenchlorid bei etwa -10 °C mit einer Lösung von Phosgen in Toluol und Reaktion des erhaltenen Steroid-3-chloroformiats in Chloroform oder Methylenchlorid mit einem Diol der Formel $HO-(CH_2)_p-OH$ in Gegenwart von Pyridin oder Triäthylamin bei etwa 5 °C.

Ein Alkoholcarbamat der Formel II, worin X z.B. eine Gruppe $-(CH_2)_p-N(T)C(O)-$ ist, kann man herstellen durch Umsetzung des entsprechenden Steroid-3-ols in Chloroform und THF mit Phenylchloroformiat in Gegenwart von Pyridin und Reaktion des erhaltenen Phenylcarbonats in Chloroform mit dem Amin der Formel $HO-(CH_2)_p-N(T)-H$.

Eine Verbindung der Formel II, worin X eine Gruppe der Formel $-CH_2CH[OCONH(T^1)]CH_2N(T^2)C(O)-$ und $T^1$ und $T^2$ Wasserstoff oder nieder-Alkyl sind, lässt sich herstellen durch Reaktion des entsprechenden 1-Deoxy-1-amino-3--O-tritylglycerins der Formel $T^2NHCH_2CHOHCH_2OC(C_6H_5)_3$ mit einem Steroid-3-chloroformiat in Methylenchlorid in Gegenwart von Kaliumhydroxyd, Umsetzung des erhaltenen Produkts in Methylenchlorid mit dem Isocyanat der Formel $O=C=N(T^1)$ bei 80 °C und Abspaltung der Tritylgruppe aus dem erhaltenen Produkt in Dioxan mittels Salzsäure bei 95 °C.

Ein Glycerinderivat der Formel II, worin X eine Gruppe $-CH_2CH(OH)CH_2-$ ist, erhält man durch Reaktion des entsprechenden 3-O-Tosylsteroids in Dioxan mit Glycerin bei 100 °C.

Ein Alkohol der Formel II mit einer Doppelbindung im Steroidteil lässt sich zum entsprechenden gesättigten Alkohol hydrieren, z.B. in Gegenwart von Pd/C in THF.

In den nachstehenden Beispielen A bis M ist die Herstellung von einigen Verbindungen der Formel II im Detail beschrieben.

## Beispiel A

Eine Lösung von 10 g Cholesterintosylat und 25,3 g Aethylenglykol in 180 ml Dioxan wird unter Rühren

auf 120° C erhitzt. Das Gemisch wird dann mit 200 ml Wasser versetzt und mit Aether extrahiert. Die ätherische Phase wird zuerst mit 10%iger Natriumcarbonatlösung und dann mit Wasser gewaschen. Die organische Phase wird getrocknet und eingedampft. Der Rückstand wird unter Eluieren mit Aether-Hexan an Kieselgel chromatographiert. Man erhält 5,6 g 2-(Cholest-5-en-3β-yloxy)-1-äthanol, Smp. 92-95° C, das Ausgangsmaterial im Beispiel 2q.

Beispiel B

Analog Beispiel A erhält man:

a) 2-[[(E)-Stigmasta-5,2-dien-3β-yl]oxy]-1-äthanol, MS: 456 ($M+H^+$)
b) 2-[2-[(E)-Stigmasta-5,22-dien-3β-yloxy]äthoxy]-1-äthanol, MS: 501 ($M+H^+$)
c) 2-[2-(Cholest-5-en-3β-yloxy)äthoxy]-1-äthanol, MS: 474 ($M+H^+$)
d) 2-[2-[2-(Cholest-5-en-3β-yloxy)äthoxy]äthoxy]äthanol, MS: 519 ($M+H^+$).

Beispiel C

1. Zu einer Lösung von 12,5 g 3-Brompropionsäurechlorid (erhalten aus 11,1 g Brompropionsäure und 9,77 ml Oxalyl chlorid in 55 ml Methylenchlorid und 3 Tropfen DMF), in 44 ml Methylenchlorid, tropft man bei 5° C eine Lösung von 20 g Stigmastanol in 200 ml Methylenchlorid und 4 ml Pyridin. Das Gemisch wird eingedampft, der Rückstand in 500 ml Methylenchlorid gelöst und diese Lösung mit verdünnter Salzsäure gewaschen. Die organische Phase wird getrocknet und eingedampft. Der Rückstand wird unter Eluieren mit Aether-Hexan an Kieselgel chromatographiert. Man erhält das 3β-Stigmastanyl-3-brompropionat vom Schmelzpunkt 150-152° C.

2. Analog werden hergestellt:

a) Stigmasta-5,22-dien-3β-yl-3-brompropionat
b) 5α-Stigmastan-3β-ylbromacetat
c) Cholest-5-en-3β-ylbromacetat
d) Cholest-5-en-3β-yl-3-brompropionat
e) 5α-Stigmastan-3β-yl-4-brombutyrat
f) Cholest-5-en-3β-yl-4-chlorbutyrat
g) 3β-Stigmastanyl-4-chlorbutyrat
h) 3β-Stigmastanyl-4-jodbutyrat
i) (E)-Stigmasta-5,22-dien-3β-yl-4-chlorbutyrat
j) (E)-Stigmasta-5,22-dien-3β-yl-4-jodbutyrat
k) Cholest-5-en-3β-yl-4-jodbutyrat
l) (E)-Stigmasta-5,22-dien-3β-yl-6-bromhexanoat
m) 5α-Stigmastan-3β-yl-6-bromhexanoat
n) Stigmast-5-en-3β-yl-4-brombutyrat.

3. Eine Lösung von 23,1 g 3β-Stigmastanyl-3-brompropionat in 400 ml Methylenchlorid und 200 ml DMF wird mit 63,3 g Kaliumtrifluoracetat versetzt und über 72 Stunden bei 80° C erhitzt. Nach Zugabe von 30 ml Wasser wird die Lösung 1 Stunde bei 80° C weitererhitzt. Die Lösung wird eingedampft und der Rückstand mittels Aether-Hexan an Kieselgel chromatographiert. Man erhält das 3β-Stigmastanyl-3-hydroxypropionat vom Schmelzpunkt 152-153° C, das Ausgangsmaterial des Beispiels 2o.

Beispiel D

Analog Beispiel C werden hergestellt:

a) Cholest-5-en-3β-ylglycolat, MS: 369 ($M-HOCH_2COOH$)
b) 3β-Stigmastanylglycolat, MS: 399 ($M-HOCH_2COOH$)
c) Cholest-5-en-3β-yl-3-hydroxypropionat, MS: 368 ($M-HOCH_2CH_2COOH$)
d) Stigmasta-5,22-dien-3β-yl-3-hydroxypropionat, MS: 394 ($M-HOCH_2CH_2COOH$)
e) Cholest-5-en-3β-yl-4-hydroxybutyrat, Smp. 98° C
f) 3β-Stigmastanyl-4-hydroxybutyrat, Smp. 149° C
g) (E)-Stigmasta-5,22-dien-3β-yl-4-hydroxybutyrat, Smp. 147° C
h) 5α-Stigmastan-3β-yl-6-hydroxyhexanoat, Smp. 130° C
i) (E)-Stigmasta-5,22-dien-3β-yl-6-hydroxyhexanoat, Smp. 133° C

j) Stigmast-5-en-3β-yl-4-hydroxybutyrat, Smp. 125-126° C.

Beispiel E

Zu einer Lösung von 13,8 g Aethylenglykol in 200 ml Methylenchlorid, tropft man bei 5° C eine Lösung von 10 g Cholesterylchloroformiat in 100 ml Methylenchlorid und 2,16 ml Pyridin. Die Lösung wird dann mit 300 ml Wasser versetzt und mit Methylenchlorid extrahiert. Die organische Phase wird mit Wasser gewaschen, getrocknet und eingedampft. Der Rückstand wird in Methylenchlorid-Aethanol umkristallisiert. Man erhält 6,4 g Cholest-5-en-3β-yl-2-hydroxyäthylcarbonat vom Schmelzpunkt 139-140° C, den Ausgangsalkohol von Beispiel 1.

Beispiel F

Analog Beispiel E werden hergestellt:
a) 5α-Stigmastan-3β-yl-2-hydroxyäthylcarbonat, Smp. 184° C
b) 2-Hydroxyäthyl-(E)-stigmasta-5,22-dien-3β-ylcarbonat, Smp. 172° C
c) Cholest-5-en-3β-yl-3-hydroxypropylcarbonat, Smp. 96° C
d) Cholest-5-en-3β-yl-4-hydroxybutylcarbonat, Smp. 107° C
e) Cholest-5-en-3β-yl-8-hydroxyoctylcarbonat, Smp. 79° C
f) 2-Hydroxyäthyl-stigmast-5-en-3β-ylcarbonat, Smp. 160° C.

Beispiel G

1. Zu einer auf -50° C gekühlten Suspension von 4,16 g Stigmastanol in 25 ml $CHCl_3$, 12,5 ml THF und 0,75 ml Pyridin wird eine Lösung von 1,71 g Phenylchloroformiat in 5 ml $CHCl_3$ getropft. Es werden dann noch 0,25 ml Pyridin zugegeben. Man lässt 30 Minuten bei tiefer Temperatur reagieren und noch 1 Stunde bei Raumtemperatur. Die Reaktionslösung wird in eine Lösung von Pyridin und wässrige $KHCO_3$ gegossen. Nach Beendigung der $CO_2$-Entwicklung wird zur Trockene eingedampft. Der Rückstand wird zwischen $CH_2Cl_2$ und $H_2O$ verteilt. Nach Kristallisation aus $CH_2Cl_2$-Pentan erhält man 4,75 g 3-Stigmastanyl-phenylcarbonat. Smp. 96° C.
2. Zu einer Lösung von 1,07 g des obigen Phenylcarbonats in 3 ml $CHCl_3$ werden 0,3 ml Aethanolamin gegeben. Man lässt 2 Tage bei Raumtemperatur reagieren. Der Ueberschuss an Reagens wird abdestilliert und das bei der Reaktion entstandene Phenol wird als Na-Phenolat abgetrennt. Die Verbindung wird aus $CH_2Cl_2$ kristallisiert. Man erhält 0,9 g 3β-Stigmastanyl-(2-hydroxyäthyl)carbamat, Smp. 206° C, das Ausgangsmaterial in Beispiel 5.

Beispiel H

Analog Beispiel G erhält man
1. aus Stigmasterin über das (E)-Stigmasta-5,22-dien-3β-yl-phenylcarbonat, Smp. 156-157° C,
a) das (E)-Stigmasta-5,22-dien-3β-yl-(2-hydroxyäthyl)carbamat, Smp. 187° C und
b) das (E)-Stigmasta-5,22-dien-3β-yl-(3-hydroxypropyl)carbamat, Smp. 172-173° C
2. aus Cholesterin über das 3β-Cholesteryl-phenylcarbonat das 3β-Cholesteryl-(2-hydroxyäthyl)carbamat, Smp. 168-170° C
3. aus Epicoprostanol über das Epicoprostanyl-phenylcarbamat das Epicoprostanyl-(2-hydroxyäthyl)-carbamat, Smp.98° C
4. aus β-Sitosterol über das Stigmast-5-en-3-yl-phenylcarbonat, Smp. 108-109° C, das Stigmast-5-en-3β-yl-(2-hydroxyäthyl)carbamat, Smp. 198-199° C, das Ausgangsmaterial in Beispiel 19.

Beispiel I

1. Einer auf -10° C gekühlten Lösung von 4,12 g Stigmasterin in 40 ml Methylenchlorid werden 10 ml einer 20%igen Phosgenlösung in Toluol zugesetzt. Man lässt über Nacht reagieren und kühlt dann auf -10° C ab. Nach Zugabe von 0,7 ml Triäthylamin in 10 ml Methylenchlorid wird die Reaktion 24 Stunden

fortgesetzt. Man neutralisiert mit 0,7 ml Triäthylamin in 10 ml Methylenchlorid und isoliert das (E)-Stigmasta-5,22-dien-3β-yl-chloroformiat.

2. Zu einer im Eisbad gekühlten Lösung von 1,6 g Aethylenglykol und 0,5 ml Pyridin in 10 ml Chloroform wird eine Lösung von 2,3 g des obigen Chloroformiats in 10 ml Chloroform getropft. Das Reaktionsgemisch wird auf Eis und eine Natriumbicarbonatlösung gegossen. Man extrahiert mit Methylenchlorid, chromatographiert an Kieselgel mit Toluol/Chloroform/Aethylacetat (4/2/1 Vol.). Nach Kristallisation aus Methylenchlorid-Methanol erhält man 1,86 g (E)-Stigmasta-5,22-dien-3β-yl-(2-hydroxyäthyl)-carbamat, Smp. 172-173 °C, den Ausgangsalkohol von Beispiel 9.

Beispiel J

1. Eine Lösung von 1,45 g 3-O-Tosylstigmasterin in 20 ml Dioxan wird 2 Stunden bei 100 °C unter Rühren mit 2,5 g Glycerin umgesetzt. Nach Abdestillieren des Lösungsmittels, Verdünnung des Rückstands mit Wasser, Extraktion mit Diäthyläther und Kristallisation aus Methylenchlorid-Methanol erhält man 1 g O-3α,β-Stigmasteryl-glycerin.

2. Eine Lösung von 1,7 g des obigen Produkts in 15 ml THF wird über 0,5 g 10%-igem Pd/C unter Normaldruck hydriert. Nach Entfernung des Katalysators, Abdestillieren des Lösungsmittels und Kristallisation aus THF-Methanol erhält man in quantitativer Ausbeute das O-3α,β-Stigmastanyl-(RS)-glycerin, das Ausgangsmaterial des Beispiel 11.

Beispiel K

1. Man lässt 0,74 g (RS)-Glycidol und 2,8 g Tritylchlorid in 5 ml Pyridin über Nacht reagieren. Dann wird unter Rühren eine Lösung von 2 g Kaliumbicarbonat zugegeben. Nach Eindampfen, Verteilung des Rückstandes zwischen Wasser und Methylenchlorid und Chromatographie an Kieselgel mit Toluol erhält man 2,25 g (RS)-Epoxy-1-O-tritylglycerin.

2. Im Bombenrohr werden 3 g dieses Produkts 2 Stunden bei 100 °C mit 5 ml Isopropylamin umgesetzt. Nach Abdestillieren des überschüssigen Isopropylamins werden aus Methylenchlorid-Hexan 4,4 g (RS)-1-Deoxy-1-isopropylamino-3-O-tritylglycerin, Smp. 128-130 °C, kristallisiert.

3. Zu einer auf -10 °C gekühlten Lösung von 1,1 g Stigmasterylchloroformiat in 4 ml Methylenchlorid werden unter Rühren 1,04 g des obigen Amins in 20 ml Methylenchlorid und 0,2 g Kaliumhydroxyd in 2 ml Wasser getropft. Nach 2-stündiger Reaktion bei Raumtemperatur, Phasentrennung im Scheidetrichter, Chromatographie an Kieselgel mit Methylenchlorid/Diäthyläther (2/1) erhält man 1,63 g (E)-Stigmasta-5,22-dien-3β-yl-isopropyl-[(RS)-2-hydroxy -3-trityloxypropyl]-carbamat, Smp. 75-76 °C.

4. Man lässt eine Lösung von 2 g des obigen Produkts in 10 ml Methylenchlorid mit 2 ml Methylisocyanat 40 Stunden bei 80 °C im Bombenrohr reagieren und erhält in quantitativer Ausbeute (E)-Stigmasta-5,22-dien-3β-yl-isopropyl-[(RS)-2-methylcarbamoyl-3-trityloxypropyl]-carbamat, Smp. 92-93 °C.

5. Durch Abspaltung der Tritylgruppe analog Beispiel 11c erhält man aus 2,5 g des obigen Trityläthers 1,7 g (E)-Stigmasta-5,22-dien-3β-yl-[(RS)-3-hydroxy -2-[(methylcarbamoyl)oxy]propyl]-isopropylcarbamat, Smp. 240 °C, das Ausgangsmaterial in Beispiel 7c.

Beispiel L

Eine Lösung von 10,2 g Cholesterylchloroformiat in 130 ml Methylenchlorid wird unter Argon unter Rühren mit 4,6 g 2-O-Benzylglycerin in 120 ml Methylenchlorid und 2 ml Pyridin versetzt, nach 1 Stunde in 500 ml Wasser und 50 ml 1N HCl aufgenommen und mit Methylenchlorid extrahiert. Die organische Phase wird getrocknet und bei 50 °C eingeengt. Nach Chromatographie über $SiO_2$ mit n-Hexan:Aether (1:1) erhält man 6,17 g (RS)-2-(Benzyloxy)-3-hydroxypropylchlorid-5-en-3β-ylcarbonat, MS: 595 ($M+H^+$), das Ausgangsmaterial des Beispiels 13.

Beispiel M

Eine Lösung von 3,86 g Cholesterin und 0,85 ml L-(+)-Milchsäure (90% in Wasser) in 80 ml Toluol wird während 1 Stunde gekocht. Das Wasser wird abgetrennt, das abgekühlte Reaktionsgemisch wird mit 0,3 g p-Toluolsulfonsäure versetzt und weitere 4 Stunden gekocht. Das Reaktionsgemisch wird mit 30 ml Wasser versetzt und mit Aether extrahiert. Die organische Phase wird über Natriumsulfat getrocknet und eingeengt. Der Rückstand wird an Silicagel (Elutionsmittel Petroläther/Aether 4:1) gereinigt. Man erhält 1,5 g

Cholest-5-en-3β-yl-(S)-2-hydroxypropionat, Smp. 120-122° C.

Die Steroide der Formel I und die Salze davon hemmen die intestinale Resorption von Cholesterin.

Die Hemmung der intestinalen Resorption von Cholesterin lässt sich tierexperimentell wie folgt nachweisen:

Totenkopfäffchen werden die zu untersuchenden Substanzen zusammen mit einer Protein, Stärke, Triolein und [26-$^{14}$C]-Cholesterin enthaltenden Testmahlzeit oral verabreicht. Hierauf wird der Kot während 2,5 Tagen gesammelt. Die im Kot ermittelte Differenz zwischen dem verabreichten und dem ausgeschiedenen radioaktiven Cholesterin wird als Mass für resorbiertes Cholesterin genommen. Die Cholesterinresorption (CHORES) wird in Prozenten der vor der Medikation ermittelten Kontrollwerte ausgedrückt.

In der nachstehenden Tabelle sind die Resultate wiedergegeben, welche mit einigen repräsentativen erfindungsgemässen Produkten erhalten wurden. Angegeben werden für jede der darin figurierenden Verbindungen die bei einer Dosis von 100 μMol/kg p.o. ermittelte Cholesterinresorption in Prozenten derjenigen in der Vorperiode. Ausserdem enthält die Tabelle Angaben über die akute Toxizität der untersuchten Verbindungen ($DL_{50}$ in mg/kg bei einmaliger oraler bzw. intravenöser Verabreichung an Mäusen).

Tabelle

| Verbindung der Formel I von Beispiel Nr. | 2a | 2k | 2q | 2r | 4a |
|---|---|---|---|---|---|
| CHORES in % der Vorperiode: | 38 | 39 | 16 | 37 | 30 |
| $DL_{50}$ in mg/kg p.p. | 4000 | 4000 | 4000 | 4000 | |
| i.v. | 250 | 250 | 250 | 500 | |
| Verbindung der Formel I von Beispiel Nr. | 5 | 6 | 7a | 16 | |
| CHORES in % der Vorperiode: | 40 | 31 | 35 | 40 | |
| $DL_{50}$ in mg/kg p.o. | | | 4000 | | |

Die erfindungsgemässen Produkte können als Heilmittel, z.B. in Form pharmazeutischer Präparate, Verwendung finden. Die pharmazeutischen Präparate werden oral, z.B. in Form von Tabletten, Lacktabletten, Dragées, Hart- und Weichgelatinekapseln, Lösungen, Emulsionen oder Suspensionen, verabreicht.

Zur Herstellung von pharmazeutischen Präparaten kann man die erfindungsgemässen Produkte mit pharmazeutisch inerten, anorganischen oder organischen Trägern vermischen. Als Träger kann man für Tabletten, Lacktabletten, Dragees und Hartgelatinekapseln beispielsweise Lactose, Maisstärke, Talk, Stearinsäure oder deren Salze verwenden. Für Weichgelatinekapseln eignen sich als Träger beispielsweise pflanzliche Oele, Wachse, Fette oder halbfeste und flüssige Polyole; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker und Glukose

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend ein Steroid der Formel I oder ein pharmazeutisch annehmbares Salz davon ebenfalls Gegenstand der vorliegenden Erfindung, weiterhin auch ein Verfahren zur Herstellung solcher Arzneimittel, welches dadurch gekennzeichnet ist, dass man eine oder mehrere erfindungsgemässe Produkte und gegebenenfalls einen oder mehrere andere therapeutisch wertvolle Stoffe in eine galenische Darreichungsform bringt. Wie eingangs erwähnt können die erfindungsgemässen Produkte bei der Bekämpfung bzw. Verhütung von Krankheiten verwendet werden.

Sie können insbesondere bei der Bekämpfung bzw. Verhütung der Hypercholesterinämie und der Atherosklerose verwendet werden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 50 mg bis etwa 3 g, vorzugsweise von etwa 200 mg bis etwa 1 g angemessen sein.

In den nachfolgenden Beispielen ist die Herstellung von Verbindungen der Formel I beschrieben.

Beispiel 1

Zu einer Lösung von 12,5 ml Phosphoroxychlorid tropft man bei Raumtemperatur eine Lösung von 48,7 g Cholest-5-en-3β-yl-2-hydroxyäthylcarbonat und 10 ml Chinolin in 500 ml Methylenchlorid. Die Lösung wird unter Rühren mit 60 ml Pyridin und 77 g Cholintosylat in 500 ml Methylenchlorid bei Raumtemperatur versetzt, worauf man das Reaktionsgemisch bei Raumtemperatur über Nacht rührt. Man versetzt mit 125 ml Wasser und 34 g Natriumbicarbonat und dann mit 3000 ml Aceton. Das ausgefallene Produkt wird abgenutscht, in 1000 ml Chloroform-Methanol 1:1 gelöst und mit 500 g Ionenaustauscher (Amberlite MB-3) gerührt. Letzterer wird abgenutscht und die Lösung wird abgedampft. Der entstandene Rückstand wird in einem Gemisch von Methylenchlorid-Methanol 1:1 und Dioxan umkristallisiert. Man erhält 39 g O-[[2-[[(Cholest-5-en-3β -yloxy)carbonyl]oxy]äthoxy]hydroxyphosphinyl]cholinhydroxid -inneres Salz vom Schmelzpunkt 224° C, MS: 640 $(M+H)^+$.

Beispiel 2

Analog Beispiel 1 erhält man ausgehend

a) von 5α-Stigmastan-3β-yl-2-hydroxyäthylcarbonat das O-[[2-[[(5α-Stigmastan-3β-yloxy)carbonyl]oxy]äthoxy]hydroxyphosphinyl]cholinhydroxid-inneres Salz, Smp. 220° C

b) von 2-Hydroxyäthyl (E)-stigmasta-5,22-dien-3β-yl-carbonat das O-[Hydroxy-[2-[[[E)-stigmasta-5,22-dien-3β-yloxy]carbonyl]äthoxy]phosphinyl]cholinhydroxid-inneres Salz, Smp. 220° C

c) von Cholest-5-en-3β-yl-3-hydroxypropylcarbonat das O-[[3-[[(Cholest-5-en-3β-yloxy)carbonyl]oxy]propoxy]hydroxyphosphinyl]cholinhydroxid-inneres Salz, Smp. 230° C

d) von Cholest-5-en-3β-yl-4-hydroxybutylcarbonat das O-[[4-[[(Cholest-5-en-3β-yloxy)carbonyl]oxy]butoxy]hydroxyphosphinyl]cholinhydroxid-inneres Salz, Smp. 232° C

e) von Cholest-5-en-3β-yl-8-hydroxyoctylcarbonat das O-[[[8-[[(Cholest-5-en-3β-yloxy)carbonyl]oxy]octyl]oxy]hydroxyphosphinyl]cholinhydroxid-inneres Salz, Smp. 235° C

f) von Cholest-5-en-3β-ylglycolat das O-[[[(Cholest-5-en-3β-yloxy)carbonyl]methoxy]hydroxyphosphinyl]cholinhydroxid-inneres Salz, Smp. 226° C

g) von Cholest-5-en-3β-yl-3-hydroxypropionat das O-[[2-[(Cholest-5-en-3β-yloxy)carbonyl]äthoxy]hydroxyphosphinyl]cholinhydroxid-inneres Salz, Smp. 180° C

h) von 3β-Stigmastanylhydroxypropionat das O-[Hydroxy-[2-[(3β-stigmastanyloxy)carbonyl]äthoxy]phosphinyl]cholinhydroxid-inneres Salz, Smp. 187° C

i) von Stigmasta-5,22-dien-3β-yl-3-hydroxypropionat das O-[Hydroxy-[2-[(stigmasta-5,22dien-3β-yloxy)carbonyl]äthoxy]phosphinyl]cholinhydroxid-inneres Salz, Smp. 199° C

j) von Cholest-5-en-3β-yl-4-hydroxybutyrat das O-[[3-[(Cholest-5-en-3β-yloxy)carbonyl]propoxy]hydroxyphosphinyl]cholinhydroxid-inneres Salz, Smp. 255° C

k) von 3β-Stigmastanyl-4-hydroxybutyrat das O-[Hydroxy-[3-[(3β-stigmastanyloxy)carbonyl]propoxy]phosphinyl]cholinhydroxid-inneres Salz, Smp. 250° C

l) von (E)-Stigmasta-5,22-dien-3β-yl-4-hydroxidbutyrat das O-[Hydroxy-[3-[(E)-stigmasta-5,22-dien-3β-yloxy)carbonyl]propoxy]phosphinyl]cholinhydroxid-inneres Salz, Smp. 225° C

m) von 5α-Stigmastan-3β-yl-6-hydroxyhexanoat das O-[Hydroxy-[[5-[(5α-stigmastan-3β-yloxy)carbonyl]pentyl]oxy]phosphinyl]cholinhydroxid-inneres Salz, Smp. 254° C

n) von (E)-Stigmasta-5,22-dien-3β-yl-6-hydroxyhexanoat das O-[Hydroxy-[[5-[((E)-stigmasta-5,22-dien-3β-yloxy)carbonyl]pentyl]oxy]phosphinyl]cholinhydroxid-inneres Salz, Smp. 215° C

o) von 3β-Stigmastanylglycolat das O-[Hydroxy-[[[(3βstigmastanyl)oxy]carbonyl]methoxy]phosphinyl]cholinhydroxid-inneres Salz, Smp. 260° C

p) von nicht isoliertem (E)-Stigmasta-5,22-dien-3β-yl-yl-glycolat das O-[Hydroxy-[[[(E)-stigmasta-5,22-dien-3β-yloxy]carbonyl]methoxy]phosphinyl]cholinhydroxid-inneres Salz, Smp. 215° C

q) von 2-(Cholest-5-en-3β-yloxy)-1-äthanol das O-[[2-(Cholest-5-en-3β-yloxy)äthoxy]hydroxyphosphinyl]cholinhydroxid-inneres Salz, MS: 596 $(M+H^+)$

r) von 2-[[(E)-Stigmasta-5,22-dien-3β-yl]oxy]-1-äthanol das O-[Hydroxy-[2-(stigmasta-5,22-dien-3β-yloxy)äthoxy]phosphinyl]cholinhydroxid-inneres Salz, Smp. 230° C

s) von 2-[2-(Cholest-5-en-3β-yloxy)äthoxy]-1-äthanol das O-[[2-[2-(Cholest-5-en-3β-yloxy)äthoxy]äthoxy]hydroxyphosphinyl]cholinhydroxid-inneres Salz, MS: 640 $(M+H^+)$

t) von 2-[2-[(E)-Stigmasta-5,22-dien-3β-yloxy]äthoxy]-1-äthanol das O-[Hydroxy-[2-[2-[(E)-stigmasta-5,22-dien-3β-yloxy]äthoxy]äthoxy]phosphinyl]cholinhydroxid-inneres Salz, MS: 666 ($M+H^+$)
u) von 2-[2-[2-(Cholest-5-en-3β-yloxy)äthoxy]äthoxy]äthanol das O-[[2-[2-[2-(Cholest-5-en-3β-yloxy)-äthoxy]äthoxy]äthoxy]hydroxyphosphinyl]cholinhydroxid-inneres Salz, MS: 684 ($M+H^+$).

## Beispiel 3

1 g O-[[2-[(Cholest-5-en-3β -yloxy)carbonyloxy]äthoxy]hydroxyphosphinyl]cholinhydroxid -inneres Salz wird in 100 ml Methanol mit 1 g Pd/C 5% unter 30 bar $H_2$ bei 80° C hydriert. Nach Filtration wird die Lösung abgedampft und der Rückstand in Chloroform-Methanol-Dioxan gelöst. Durch Zugabe von Aether erhält man 0,6 g O-[[2-[(5α-Cholestan-3β-yloxy)carbonyloxy]äthoxy]hydroxyphosphinyl]cholinhydroxid-inneres Salz als hygroskopisches Pulver, Smp. 225° C.

## Beispiel 4

In zu Beispiel 3 analoger Weise erhält man unter Verwendung
a) von O-[Hydroxy-[2-[2-[(E)-stigmasta-5,22-dien-3β-yloxy]äthoxy]äthoxy]phosphinyl]cholinhydroxid-inneres Salz das O-[Hydroxy-[2-[2-(3β-stigmastanyloxy)äthoxy]äthoxy]phosphinyl]cholinhydroxid-inneres Salz, MS: 670 ($M+H^+$)
b) von O-[Hydroxy-[2-(stigmasta-5,22-dien-3β-yloxy)äthoxy]phosphinyl]cholinhydroxid-innerem Salz das O-[Hydroxy-[2-(3β-stigmastanyloxy)äthoxy]phosphinyl]cholinhydroxid-inneres Salz, Smp. 200° C.

## Beispiel 5

1 g 3β-Stigmastanyl-(2-hydroxyäthyl)-carbamat und 0,35 ml $Et_3N$ werden in 5 ml THF gelöst. Zu der im Eisbad gekühlten Lösung wird eine Lösung von 315 mg 2-Chlor-2-oxo-1,3,2-dioxaphospholan in 3 ml THF getropft. Die erhaltene Suspension wird während 5 Stunden bei Raumtemperatur gerührt. Dann wird der $Et_3N$•HCl-Niederschlag abfiltriert und die zurückbleibende Lösung wird eingedampft.

Das erhaltene Phosphat wird in 10 ml THF gelöst, die Lösung wird im Druckkolben mit 1 g $(CH_3)_3N$ in 10 ml THF versetzt. Man lässt bei 70° C während 24 Stunden reagieren. Nach Abdestillieren des überschüssigen Reagens und des Lösungsmittels wird der Rückstand in 20 ml MeOH-$CHCl_3$ aufgenommen und die Lösung wird durch einen Ionenaustauscher (Amberlite MB3) perkoliert. Das Produkt wird an Kieselgel mit $CHCl_3$-MeOH-$H_2O$ (60/35/5 in Vol.) chromatographiert. Man erhält 0,6 g O-[Hydroxy-[2-[1-(5α-stigmastan-3β-yloxy)formamido]äthoxy]phosphinyl]cholinhydroxid-inneres Salz, MS: 669 ($M+H^+$).

## Beispiel 6

Die Herstellung des Phosphats wird wie in Beispiel 5 beschrieben wiederholt. Das aus 2 mM Ausgangscarbamat erhaltene Material wird in 5 ml trockenem Pyridin gelöst und man lässt bei 80° C während 24 Stunden reagieren. Das Produkt wird analog Beispiel 5 isoliert und gereinigt. Man erhält 0,95 g 1-[2-[[Hydroxy-[2-[1-(5α-stigmastan-3β -yloxy)formamido]äthoxy]phosphinyl]oxy]äthyl] pyridiniumhydroxid-inneres Salz, MS: 689 ($M+H^+$).

## Beispiel 7

Analog Beispiel 5 erhält man ausgehend
a) von (E)-Stigmasta-5,22-dien-3β-yl-(2-hydroxyäthyl)carbamat das O-[Hydroxy-[2-[1[(E)-stigmasta-5,22-dien-3β-yloxy]formamido]äthoxy]phosphinyl]cholinhydroxid-inneres Salz, MS: 665 ($M+H^+$)
b) von (E)-Stigmasta-5,22-dien-3β-yl-(3-hydroxypropyl)carbamat das O-[Hydroxy-[3-[1-[(E)-stigmasta-5,22-dien-3β-yloxy]formamido]propoxy]phosphinyl]cholinhydroxid-inneres Salz, MS: 679 ($M+H^+$)
c) von (E)-Stigmasta-5,22-dien-3β-yl-[(RS)-3-hydroxy-2-[(methylcarbamoyl)oxy]propyl]-isopropylcarbamat das O-[Hydroxy-[(RS)-3-[N-isopropyl-1-[(E)-stigmasta-5,22-dien3β-yl]oxyformamido]-2-[-(methylcarbamoyl)oxy]propoxy]phosphinyl]cholinhydroxid-inneres Salz, MS: 794 ($M+H^+$)

d) von 3β-Cholesteryl-(2-hydroxyäthyl)carbamat das O-[[[2-[1-Cholest-5-en-3β-yloxy]formamido]äthoxy]-hydroxyphosphinyl]cholinhydroxid-inneres Salz, MS: 639 ($M+H^+$)
e) von Epicoprostanyl-(2-hydroxyäthyl)carbamat das O-[[2-[1-(5β-Cholestan-3α-yloxy)formamido]äthoxy]-hydroxyphosphinyl]cholinhydroxid-inneres Salz, MS: 641 ($M+H^+$).

## Beispiel 8

Analog Beispiel 6 erhält man ausgehend von (E)-Stigmasta-5,22-dien-3β-yl-(3-hydroxypropyl)carbamat das 1-[2-[[Hydroxy-[3-[1-[(E)-stigmasta-5,22-dien-3β-yloxy]-formamido]propoxy]phosphinyl]oxy]äthyl]-pyridiniumhydroxid-inneres Salz, MS: 699 ($M+H^+$).

## Beispiel 9

Eine Lösung von 2,22 g (E)-Stigmasta-5,22-dien-3β-yl-(2-hydroxyäthyl)carbonat und 1,1 ml Pyridin in 10 ml Chloroform wird einer auf 0 °C abgekühlten Lösung von 0,75 g Phosphoroxychlorid in 3 ml Chloroform zugetropft. Man lässt 1 Stunde bei Raumtemperatur reagieren. Dann werden 1,6 g Cholintosylat in 15 ml Pyridin zugeführt. Es wird über Nacht gerührt, dann eine Lösung von 2 g Kaliumbicarbonat zugesetzt. Das Gemisch wird zur Trockene eingedampft und der Rückstand in 100 ml THF/Methanol/Chloroform (1/1/1) aufgenommen. Die Feststoffe werden abfiltriert. Die zurückbleibende Lösung wird über einem Ionenaustauscher (Amberlite MB-3) perkoliert. Nach Chromatographie an Kieselgel mit Chloroform/Methanol/Wasser (60/35/5 Vol) und Kristallisation aus Methylenchlorid/Aceton erhält man 1,6 g O-[Hydroxy-[2-[[[(E)-stigmasta-5,22-dien-3β-yloxy]carbonyl]oxy]äthoxy]phosphinyl]cholinhydroxid -inneres Salz, MS: 666 ($M+H^+$).

## Beispiel 10

4,75 g 3β-Cholesteryl-(2-hydroxyäthyl)carbamat werden mit 2-Chlor-2-oxo-1,3,2-dioxaphospholan umgesetzt. Das erhaltene Phosphat wird bei 70 °C 24 Stunden mit N-Methylpyrrolidon versetzt. Nach Chromatographie an Kieselgel mit Chloroform/Methanol/Wasser (30/62,5/7,5 Vol.) erhält man 1,2 g 1-[2-[[[2-[1-[Cholest-5-en-3β-yloxy]formamido]äthoxy]hydroxyphosphinyl]oxy]äthyl] -1-methylpyrrolidiniumhydroxid-inneres Salz, MS: 665 ($M+H^+$).

## Beispiel 11

a) Einer Lösung von 2,49 g O-3α,β-Stigmastanyl-(RS)-glycerol in 10 ml Pyridin werden 1,41 g Tritylchlorid zugesetzt. Nach 48 Stunden Reaktion wird unter Rühren eine Kaliumbicarbonatlösung zugegeben. Es wird zur Trockene eingedampft. Der Rückstand wird zwischen Methylenchlorid und Wasser verteilt. Nach Chromatographie an Kieselgel mit Methylenchlorid erhält man das 1-O-Trityl-3-O-(3α,β-stigmastanyl)-(RS)-glycerin.
b) Einer Suspension von 96 mg Natriumhydrid in 5 ml THF werden 2,4 g des obigen Trityläthers in 10 ml THF und dann 0,46 ml Benzylchlorid in 5 ml DMF zugetropft. Nach 2 Stunden Reaktion bei 80 °C, Abdestillieren des Lösungsmittels und Verteilung des Rückstands zwischen Methylenchlorid und Wasser erhält man 1-O-Trityl-2-O-benzyl-3-O-(3α,β-stigmastanyl) -(RS)-glycerin.
c) Einer auf 95 °C erwärmten Lösung von 2,4 g des obigen Benzyläthers in 20 ml Dioxan werden 2 ml 1N Salzsäure gegeben. Nach 2 Stunden Reaktion bei 95 °C wird das Lösungsmittel abdestilliert, der Rückstand in Hexan aufgenommen und das ausgefallene Triphenylmethanol abgetrennt. Nach Chromatographie an Kieselgel mit Toluol/Aethylacetat (9/1) erhält man 1,5 g 1-O-(3α,β-Stigmastanyl)-2-O-benzyl-(RS)-glycerin.
d) Analog Beispiel 5 erhält man aus 1,4 g des obigen Glycerolderivates zunächst durch Reaktion mit 2-Chlor-2-oxo-1,3,2-dioxaphospholan und dann Umsetzung des erhaltenen Phosphats mit Trimethylamin 0,8 g 1-O-(3α,β-Stigmastanyl)- 2-O-benzyl-3-O-(RS) -glyceryl-phosphorylcholin, MS: 746 ($M+H^+$).
e) Eine Lösung von 0,77 g des obigen Benzyläthers in 10 ml Methanol und 5 ml THF wird unter Normaldruck in Gegenwart von 10% Pd/C hydriert. Man erhält in quantitativer Ausbeute das 1-O-(3α,β-Stigmastanyl)-3-O-(RS)-glyceryl-phosphorylcholin, MS: 656 ($M+H^+$).

## Beispiel 12

Einer Lösung von 0,5 g des Glycerinderivates des Beispiels 11 in 3 ml Chloroform und 1 ml Pyridin werden 50 mg DMAP und 0,5 ml Essigsäureanhydrid zugefügt. Nach 1 Stunde Reaktion wird zur Trockene eingeengt. Die Lösung des Rückstands in 20 ml Methanol wird über Ionenaustauscher (Amberlite MB-3) perkoliert. Man erhält 0,51 g O-[[(RS)-2-Acetoxy-3-[5a-stigmastan-3α,β -yloxy]propoxy]hydroxyphosphonyl]-cholinhydroxid-inneres Salz, MS: 698 ($M+H^+$).

Beispiel 13

a) 1,15 ml Phosphoroxychlorid werden unter Argon und Rühren mit 6,1 g (RS)-2-(Benzyloxy)-3-hydroxypropylcholest-5-en-3β-ylcarbonat und 1,5 ml Chinolin in 80 ml Methylenchlorid versetzt. Nach 4 Stunden gibt man 7,4 g Cholintosylat und 6 ml Pyridin zu und nach 24 Stunden 15 ml Wasser und 4 g $NaHCO_3$. Nach 30 Minuten wird das Gemisch auf 500 ml Wasser und 50 ml 1N HCl gegossen und mit Chloroform extrahiert. Die organische Phase wird eingeengt. Das Produkt wird in 150 ml MeOH-$CHCl_3$ - (2:1) gelöst und mit 100 g Ionenaustauscher (Amberlit MB3) versetzt. Nach 16 Stunden Rühren wird der Ionenaustauscher abgenutscht. Die erhaltene Lösung wird bei 60 °C eingeengt, mit Toluol destilliert und getrocknet. Der Rückstand wird über Kieselgel mit $CHCl_3$-MeOH (1:1) und $CHCl_3$-MeOH-$H_2O$ (60:35:5) gereinigt. Man erhält nach Trocknung 4,9 g O-[[(RS)-2-Benzyloxy-3-[(cholest-5-en- -3β -yloxy)-carbonyloxy]propoxy]hydroxyphosphinyl]cholinhydroxid -inneres Salz, Ausbeute: 63%. MS: 760 ($M+H^+$).

b) 4,65 g des Produkts von a) und 1,4 g PdO in 200 ml $CH_3COOH$ werden unter $H_2$ unter Normaldruck 20 Minuten gerührt. Nach Abfiltrieren des Katalysators wird die Lösung bei 60 °C eingeengt. Der Rückstand wird in 200 ml Aether gewaschen, filtriert und getrocknet. Der Rückstand wird in 40 ml $CHCl_3$-MeOH (1:1) gelöst und mit 25 ml Dioxan und 200 ml Aether versetzt. Die ausgefallenen Kristalle werden abgenutscht, mit Aether gewaschen und bei 50 °C getrocknet. Man erhält 3,25 g O-[[(RS)-3-[-(cholest-5-en-3β-yloxy)carbonyloxy] -2-hydroxypropoxy]hydroxyphosphinyl]cholinhydroxid-inneres Salz (stark hygroskopisch), Ausbeute: 79%, MS: 670 ($M+H^+$).

Beispiel 14

Analog Beispiel 13 lassen sich herstellen:

a) das O-[Hydroxy-(RS)-2-hydroxy-3-[[5α-stigmastan-3β-yloxy)carbonyloxy]propoxy]phosphinyl]-cholinhydroxid-inneres Salz (Epimere 1:1), MS: 700 ($M+H^+$)

b) das O-[Hydroxy-[(RS)-2-hydroxy-3-[[(E)-stigmasta-5,22-dien-3β-yloxy]carbonyloxy]propoxy]-phosphinyl]cholinhydroxid-inneres Salz (Epimere 1:1), MS: 696 ($M+H^+$).

Beispiel 15

a) Zu einer Lösung von 1 g β-Bromäthyl-phosphorsäuredichlorid in 10 ml Methylenchlorid tropft man eine Lösung von 1 g Cholest-5-en-3β-yl-(5)-2-hydroxypropionat und 0,37 ml Triäthylamin in 5 ml Methylenchlorid. Man rührt das Gemisch zunächst 4 Stunden bei Raumtemperatur, dann 1 Stunde unter Rückfluss. Nach Zugabe von 5 ml Wasser wird das Gemisch während 2 Stunden am Rückfluss gekocht. Man verdünnt mit Methylenchlorid, trennt die wässrige Phase ab, wäscht die organische Phase mit Wasser, trocknet über Natriumsulfat und engt ein. Der Rückstand wird in Aether gelöst und mit einer Lösung von 5 g Bariumacetat in 20 ml Wasser versetzt. Man rührt während 20 Stunden bei Raumtemperatur, filtriert das entstandene Bariumsalz ab und versetzt dieses mit einem Gemisch von 20 ml 3N Salzsäure und 20 ml Methylenchlorid. Man rührt 1 Stunde bei Raumtemperatur, trennt die organische Phase ab, trocknet über Natriumsulfat und engt ein. Man erhält 0,7 g Cholest-5-en-3β-yl-(S) -2-[[(2-bromäthoxy)hydroxyphosphinyl]oxy]propionat, MS: 643 $(M-H)^-$.

b) Eine Lösung von 0,7 g Cholest-5-en-3β-yl-(S)-[[(2-bromäthoxy)hydroxyphosphinyl)oxy]propionat in 15 ml Toluol wird im Trockeneisbad mit 15 ml Trimethylamin versetzt. Das Gemisch wird während 30 Stunden auf 60 °C erwärmt. Nach dem Abkühlen wird das Reaktionsgemisch in Toluol aufgenommen und eingeengt. Der Rückstand wird in 30 ml Methanol aufgenommen. mit 2 g Silbercarbonat versetzt und 0,5 Stunden bei 50 °C gerührt. Man filtriert den Niederschlag ab, engt ein und erhält 0,6 g O-[[(S)-1-[(Cholest-5-en-3β -yloxy)carbonyl]äthoxy]hydroxyphosphinyl]cholinhydroxid -inneres Salz, MS: 624 ($M+H^+$).

Beispiel 16

Analog Beispiel 1 erhält man ausgehend von Stigmast-5-en-3β-yl-2-hydroxyäthylcarbonat das O-[Hydroxy-[2-[[(stigmast-5-en-3β-yloxy)carbonyl]oxy]äthoxy]phosphinyl]cholinhydroxid-innere Salz, Smp. 207° C (Zersetzung).

Beispiel 17

Analog Beispiel 2k bzw. Beispiel 2q erhält man

a) O-[Hydroxy-[3-[(stigmast-5-en-3β-yloxy)carbonyl]propoxy]phosphinyl]cholinhydroxid-inneres Salz, Smp. 220° C (Zersetzung) bzw.

b) O-[Hydroxy-[2-(stigmast-5-en-3β-yloxy)äthoxy]phosphinyl]cholinhydroxid-inneres Salz, Smp. 197° C (Zersetzung).

Beispiel 18

Analog Beispiel 5 jedoch unter Verwendung von Dimethylamin anstatt Triäthylamin erhält man das Cholest-5-en-3β-yl-2-[[[2-(dimethylamino)äthoxy]hydroxyphosphinyl]oxy]äthylcarbonat, Smp. 150° C (Zersetzung).

Beispiel 19

Analog Beispiel 5 erhält man das O-[Hydroxy-[2-[1-(stigmast-5-en-3β-yloxy)formamido]äthoxy]-phosphinyl]cholinhydroxid-innere Salz, Smp. 225° C (Zersetzung).

Beispiel 20

Analog Beispiel 12 erhält man das O-[[(RS)-2-Acetoxy-3-[[(cholest-5-en-3β-yloxy)carbonyl]oxy]propoxy]-hydroxyphosphinyl]cholinhydroxid-innere Salz, Smp. 130° C (Zersetzung).

Beispiel 21

a) Eine Lösung von 11,6 g Cholesterin und 3,6 g Hydroxypivalinsäure in 80 ml Toluol wird mit 1 g p-Toluolsulfonsäure versetzt und während 4 Stunden gekocht. Das Reaktionsgemisch wird mit Wasser versetzt und mit Aether extrahiert. Die organische Phase wird getrocknet und eingeengt und der Rückstand über Kieselgel mit Petroläther/Aether (4:1) gereinigt. Man erhält 1,9 g Cholest-5-en-3β-yl-3-hydroxy-2,2-dimethyl-propionat, Smp. 174-176° C.

b) Zu einer Lösung von 1,9 g des Produkts von a) in 60 ml Methylenchlorid tropft man zunächst 1,1 ml Triäthylamin, dann eine Lösung von 1,42 g β-Bromäthyl-phosphorsäure-dichlorid in 30 ml Methylenchlorid. Das Gemisch wird während 18 Stunden und nach Zugabe von Wasser während 1 Stunde unter Rückfluss gekocht. Man trennt die wässrige Phase ab, wäscht die organische Phase mit Wasser, trocknet und engt ein. Der Rückstand wird in Aether gelöst und mit einer Lösung von 15 g Bariumacetat in 30 ml Wasser versetzt. Man rührt 20 Stunden, filtriert das entstandene Bariumsalz ab und versetzt dieses mit einem Gemisch von 50 ml 3N Salzsäure und 50 ml Methylenchlorid. Man rührt 1 Stunde bei Raumtemperatur, trennt die organische Phase ab, trocknet und engt ein. Man erhält 1,6 g Cholest-5-en-3β-yl-3-[[(2-bromäthoxy)hydroxy-phosphinyl]oxy]-2,2-dimethylpropionat, Smp. 98° C.

c) Eine Lösung von 1,6 g des Produkts von b) in 15 ml Toluol wird unter Kühlung im Trockeneisbad mit 15 ml Trimethylamin versetzt. Das Gemisch wird während 2 Tagen auf 60° C erwärmt. Nach Abkühlen im Trockeneisbad wird das Reaktionsgemisch mit Toluol aufgenommen und eingeengt. Der Rückstand wird in Methanol aufgenommen, mit 3 g Silbercarbonat versetzt und 0,5 Stunden bei 50° C gerührt. Man filtriert den Niederschlag ab und engt ein. Durch Umkristallisation des Rückstandes aus Aether erhält man 0,8 g (52%) O-[[2-[Cholest-5-en-3β-yloxy)carbonyl]-2-methylpropoxy]hydroxyphosphinyl]-cholinhydroxid-inneres Salz, Smp. 210° C.

Beispiel 22

a) Eine Lösung von 5,00 g Cholesterylchloroformat in 20 ml Methylenchlorid wird einem Gemisch von 1,4 g DL-Serinol•HCl und 3,1 ml Triäthylamin in 30 ml Methylenchlorid zugetropft. Nach 45 Stunden Rühren wird das Gemisch in wässrigem Methanol aufgenommen und mit Chloroform extrahiert. Die organische Phase wird getrocknet und eingeengt. Nach Chromatographie über $SiO_2$ mit Chloroform und dann mit Aether erhält man 4,3 g Cholest-5-en-3β-yl-[2-hydroxy-1-(hydroxymethyl)äthyl]carbamat, Smp. 158° C (Zersetzung).

b) Eine Lösung von 3,7 g des Produkts von a) in 30 ml Methylenchlorid, 10 ml THF und 0,6 ml Pyridin wird unter Argon mit 1 ml Phenylchloroformat in 15 ml Methylenchlorid versetzt. Nach 1 Stunde Rühren wird die Lösung in 200 ml Wasser und 50 ml HCl 0,1N aufgenommen und mit 150 ml Methylenchlorid extrahiert. Die organische Phase wird getrocknet und eingeengt. Nach Chromatographie über $SiO_2$ mit n-Hexan/Aether (1:1) und Aether erhält man 2,1 g Cholest-5-en-3β-yl-[(RS)-2-hydroxy-1-[-(phenoxycarbonyl)oxymethyl]äthyl]carbamat.

c) Einem Gemisch von 320 mg des Produkts von b) in 20 ml Toluol und 0,08 ml Triäthylamin werden unter Argon unter Rühren bei Raumtemperatur 0,05 ml 2-Chlor-2-oxo-1,3,2-dioxaphospholan zugegeben. Nach 16 Stunden wird das $(Et)_3N$:HCl abgenutscht. Die erhaltene Lösung wird eingeengt und der Rückstand in einer Lösung von 2 g Trimethylamin in 20 ml Toluol und 5 ml Acetonitril gelöst und bei 80° C behalten. Nach 6 Stunden wird die Lösung eingeengt. Das Produkt, O-[[(RS)-2-[1-(Cholest-5-en-3β-yloxy)formamido]-3-(phenoxycarbonyloxy)propoxy]hydroxyphosphinyl]cholinhydroxid-inneres Salz, wird ohne Reinigung in die nächste Stufe eingesetzt.

d) Das Produkt von c) wird in 20 ml Methanol-Chloroform (1:1) gelöst und unter Rühren bei Raumtemperatur mit 2 ml NaOH 1N versetzt. Nach Zugabe von 10 g Ionenaustauscher (Amberlite MB-3) und 4 Stunden Rühren wird die Lösung eingeengt, mit Toluol azeotropisch destilliert und dann getrocknet. Der Rückstand wird an Kieselgel mit $CHCl_3$-MeOH-$H_2O$ (60/35/5 in Vol.) chromatographiert. Nach Trocknen erhält man 200 mg O-[[(RS)-2-[1-(Cholest-5-en-3β-yloxy)formamido]-3-hydroxypropoxy]-hydroxyphosphinyl]cholinhydroxid-innere Salz, Smp. 220° C (Zersetzung).

In an sich bekannter Weise werden Tabletten und Kapseln folgender Zusammensetzung hergestellt:

Beispiel a

| Tabletten | 1 Tablette enthält |
|---|---|
| Verbindung der Formel I | 200 mg |
| Mikrokristalline Cellulose | 155 mg |
| Maisstärke | 25 mg |
| Talk | 25 mg |
| Hydroxypropylmethylcellulose | 20 mg |
| | 425 mg |

Beispiel b

| Kapseln | 1 Kapsel enthält |
|---|---|
| Verbindung der Formel I | 100,0 mg |
| Maisstärke | 20,0 mg |
| Milchzucker | 95,0 mg |
| Talk | 4,5 mg |
| Magnesiumstearat | 0,5mg |
| | 200,0 mg |

**Ansprüche**

1. Steroide der Formel

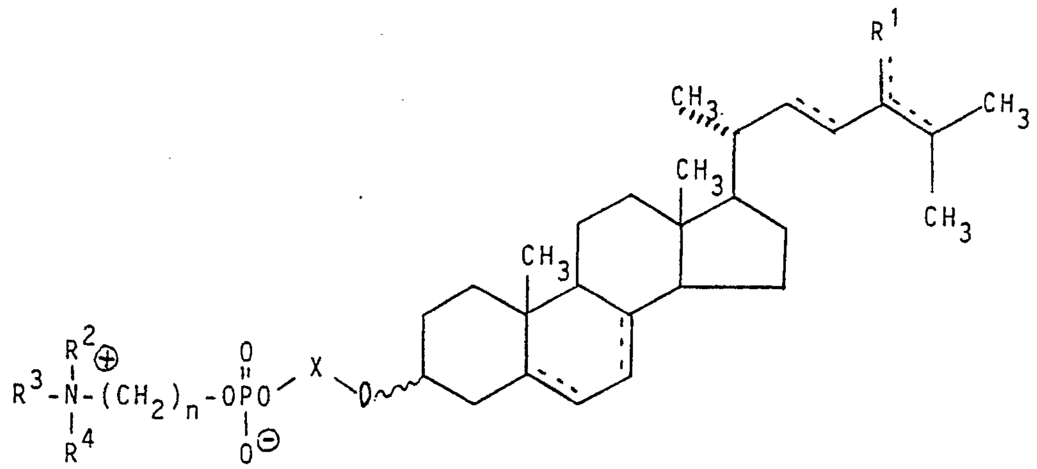

I

worin
$R^1$ Wasserstoff, nieder-Alkyl oder nieder-Alkyliden,
$R^2$, $R^3$ und $R^4$ Wasserstoff oder nieder-Alkyl sind oder zusammen mit dem N-Atom einen 5- oder 6-gliedrigen, gegebenenfalls niederalkylierten, aromatischen oder gesättigten heterocyclischen Rest bilden,
n die Zahl 2, 3 oder 4,
X eine Gruppe der Formel
$-(CH_2)_p-C(Q,Q')-(Z)_{1\ oder\ 0}-$,
$-CH_2CH(Y)CH_2-(Z)_{1\ oder\ 0}-$,
$-CH_2CH(CH_2Y)-(Z)_{1\ oder\ 0}-$ oder
$-(CH_2CH_2O)_q-CH_2CH_2-(Z)_{1\ oder\ 0}-$,
q die Zahl 1 oder 2,
Z eine eine Gruppe der Formel -C(O)-, -OC(O)-, $-OC(O)CH_2-$, $-OCH_2C(O)-$ oder -N(T)C(O)-,
Q, Q' und T Wasserstoff oder nieder-Alkyl,
p eine ganze Zahl zwischen 1 und 9 ist und, falls Z Carbonyl ist, auch 0 sein kann,
Y Hydroxy, nieder-Alkoxy, nieder-Alkanoyloxy, Carbamoyloxy oder Mono- oder Di-niederalkyl-carbamoyloxy ist, die gestrichelten C-C-Bindungen in 5(6)-, 7(8)-, 22(23)-, 24(25)- und 24(28)-Stellung fakultativ sind, wobei die Seitenkette entweder gesättigt oder einfach ungesättigt ist,
und physiologisch verträgliche Salze davon.

2. Steroide nach Anspruch 1, worin $R^1$ Wasserstoff oder nieder-Alkyl, insbesondere Aethyl, ist.

3. Steroide nach Anspruch 1 oder 2, worin $R^2$ Wasserstoff oder nieder-Alkyl und $R^3$ und $R^4$ nieder-Alkyl sind.

4. Steroide nach Anspruch 1 oder 2, worin $R^2$, $R^3$ und $R^4$ Methyl sind.

5. Steroide nach Anspruch 1 oder 2, worin $R^2$ , $R^3$ und $R^4$ zusammen mit dem N-Atom eine Pyridinium- oder 1-Methylpyrrolidiniumgruppe bilden.

6. Steroide nach einem der Ansprüche 1-5, worin n die Zahl 2 ist.

7. Steroide nach einem der Ansprüche 1-6 mit gesättigtem oder 5(6)-ungesättigtem oder 5(6)- und 22(23)-ungesättigtem Steroidanteil.

8. Steroide nach einem der Ansprüche 1-7, worin X die Gruppe $-(CH_2)_2-$, $-(CH_2)_2O(CH_2)_2-$,
$-(CH_2)_2O(CH_2)_2O(CH_2)_2-$, $-CH_2CHOHCH_2-$,
$-CH_2CH(OCOCH_3)CH_2-$, $-CH_2CH(OCONHCH_3)CH_2N[CH(CH_3)_2]CO-$,
$-(CH_2)_2NHCO-$, $-(CH_2)_3NHCO-$, $-CH_2CHOHCH_2OCO-$,
$-CH(CH_3)CO-$ oder insbesondere $-(CH_2)_2OCO-$, $-(CH_2)_3OCO-$,
$-(CH_2)_4OCO-$, $-(CH_2)_8OCO-$, $-(CH_2)CO-$, $-(CH_2)_2CO-$,
$-(CH_2)_3CO-$, $-(CH_2)_5CO-$, $-CH_2CH(OCOCH_3)CH_2OCO-$,
$-CH_2C(CH_3)_2CO-$ oder $-CH_2CH(CH_2OH)NHCO-$ darstellt.

9. Eine Verbindung nach Anspruch 1 aus der Gruppe der folgenden:
O-[[2-[[(Cholest-5-en-3β-yloxy)carbonyl]oxy]äthoxy]hydroxyphosphinyl]cholinhydroxid-inneres Salz,

O-[Hydroxy-[3-[(3β-stigmastanyloxy)carbonyl]propoxy]phosphinyl]cholinhydroxid-inneres Salz,
O-[[2-(Cholest-5-en-3β-yloxy)äthoxy]hydroxyphosphinyl]cholinhydroxid-inneres Salz und
O-[Hydroxy-[2-[1-(5α-stigmastan-3β-yloxy)formamido]äthoxy]phosphinyl]cholinhydroxid-inneres Salz.

10. Eine Verbindung nach Anspruch 1 aus der Gruppe der folgenden:
O-[[2-[[(5α-Stigmastan-3β-yloxy)carbonyl]oxy]äthoxy]hydroxyphosphinyl]cholinhydroxid-inneres Salz,
O-[Hydroxy-[2-[[[E)-stigmasta-5,22-dien-3β-yloxy]carbonyl]äthoxy]phosphinyl]cholinhydroxid-inneres Salz,
O-[[3-[[(Cholest-5-en-3β-yloxy)carbonyl]oxy]propoxy]hydroxyphosphinyl]cholinhydroxid-inneres Salz,
O-[[4-[[(Cholest-5-en-3β-yloxy)carbonyl]oxy]butoxy]hydroxyphosphinyl]cholinhydroxid-inneres Salz,
O-[[[8-[[(Cholest-5-en-3β-yloxy)carbonyl]oxy]octyl]oxy]hydroxyphosphinyl]cholinhydroxid-inneres Salz,
O-[[[(Cholest-5-en-3β-yloxy)carbonyl]methoxy]hydroxyphosphinyl]cholinhydroxid-inneres Salz,
O-[[2-[(Cholest-5-en-3β-yloxy)carbonyl]äthoxy]hydroxyphosphinyl]cholinhydroxid-inneres Salz,
O-[Hydroxy-[2-[(3β-stigmastanyloxy)carbonyl]äthoxy]phosphinyl]cholinhydroxid-inneres Salz,
O-[Hydroxy-[2-[(stigmasta-5,22-dien-3β-yloxy)carbonyl]äthoxy]phosphinyl]cholinhydroxid-inneres Salz,
O-[[3-[(Cholest-5-en-3β-yloxy)carbonyl]propoxy]hydroxyphosphinyl]cholinhydroxid-inneres Salz,
O-[Hydroxy-[3-[(E)-stigmasta-5,22-dien-3β-yloxy)carbonyl]propoxy]phosphinyl]cholinhydroxid-inneres Salz,
O-[Hydroxy-[[5-[(5α-stigmastan-3β-yloxy)carbonyl]pentyl]oxy]phosphinyl]cholinhydroxid-inneres Salz,
O-[Hydroxy-[[5-[((E)-stigmasta-5,22-dien-3β-yloxy)carbonyl]pentyl]oxy]phosphinyl]cholinhydroxid-inneres Salz,
O-[Hydroxy-[[[(3β-stigmastanyl)oxy]carbonyl]methoxy]phosphinyl)cholinhydroxid-inneres Salz,
O-[Hydroxy-[[[(E)-stigmasta-5,22-dien-3β-yloxy]carbonyl]methoxy]phosphinyl]cholinhydroxid-inneres Salz,
O-[Hydroxy-[2-(stigmasta-5,22-dien-3β-yloxy)äthoxy]phosphinyl]cholinhydroxid-inneres Salz,
O-[[2-[2-(Cholest-5-en-3β-yloxy)äthoxy]äthoxy]hydroxyphosphinyl]cholinhydroxid-inneres Salz,
O-[Hydroxy-[2-[2-[(E)-stigmasta-5,22-dien-3β-yloxy]äthoxy]äthoxy)phosphinyl]cholinhydroxid-inneres Salz,
O-[[2-[2-[2-(Cholest-5-en-3β-yloxy)äthoxy]äthoxy]äthoxy]hydroxyphosphinyl]cholinhydroxid-inneres Salz,
O-[[2-[(5α-Cholestan-3β-yloxy)carbonyloxy]äthoxy]hydroxyphosphinyl]cholinhydroxid-inneres Salz,
O-[Hydroxy-[2-[2-(3β-stigmastanyloxy)äthoxy]äthoxy]phosphinyl)cholinhydroxid-inneres Salz,
O-[Hydroxy-[2-(3β-stigmastanyloxy)äthoxy]phosphinyl]cholinhydroxid-inneres Salz,
1-[2-[[Hydroxy-[2-[1-(5α-stigmastan-3β-yloxy)formamido]äthoxy]phosphinyl]oxy]äthyl]pyridiniumhydroxid-inneres Salz,
O-[Hydroxy-[2-[1-[(E)-stigmasta-5,22-dien-3β-yloxy]formamido]äthoxy]phosphinyl]cholinhydroxid-inneres Salz,
O-[Hydroxy-[3-[1-[(E)-stigmasta-5,22-dien-3β-yloxy]formamido]propoxy]phosphinyl]cholinhydroxid-inneres Salz,
O-[Hydroxy-[(RS)-3-[N-isopropyl-1-[(E)-stigmasta-5,22-dien-3β-yl]oxyformamido]-2-[(methylcarbamoyl)oxy]-propoxy]phosphinyl]cholinhydroxid-inneres Salz,
O-[[[2-[1-Cholest-5-en-3β-yloxy]formamido]äthoxy]hydroxyphosphinyl]cholinhydroxid-inneres Salz,
O-[[2-[1-(5β-Cholestan-3α-yloxy)formamido]äthoxy]hydroxyphosphinyl]cholinhydroxid-inneres Salz,
1-[2-[[Hydroxy-[3-[1-[(E)-stigmasta-5,22-dien-3β-yloxy]formamido]propoxy]phosphinyl]oxy]äthyl]-pyridiniumhydroxid-inneres Salz,
O-[Hydroxy-[2-[[[(E)-stigmasta-5,22-dien-3β-yloxy]carbonyl]oxy]äthoxy]phosphinyl]cholinhydroxid-inneres Salz,
1-[2-[[[2-[1-[Cholest-5-en-3β-yloxy]formamido]äthoxy]hydroxyphosphinyl]oxy]äthyl]-1-methylpyrrolidiniumhydroxid-inneres Salz,
1-O-(3α,β-Stigmastanyl)-3-O-(RS)-glyceryl-phosphorylcholin,
O-[[(RS)-2-Acetoxy-3-[5α-stigmastan-3α,β-yloxy]propoxy]hydroxyphosphonyl]cholinhydroxid-inneres Salz,
O-[[(RS)-3-[(cholest-5-en-3β-yloxy)carbonyloxy]-2-hydroxypropoxy]hydroxyphosphinyl]cholinhydroxid-inneres Salz,
O-[Hydroxy-(RS)-2-hydroxy-3-[[5α-stigmastan-3β-yloxy)carbonyloxy]propoxy]phosphinyl]cholinhydroxid-inneres Salz,
O-[Hydroxy-[(RS)-2-hydroxy-3-[[(E)-stigmasta-5,22-dien-3β-yloxy]carbonyloxy]propoxy]phosphinyl]-cholinhydroxid-inneres Salz,
O-[Hydroxy-[2-[[(stigmast-5-en-3β-yloxy)carbonyl]oxy]äthoxy]phosphinyl]cholinhydroxid-inneres Salz,
O-[Hydroxy-[3-[(stigmast-5-en-3β-yloxy)carbonyl)propoxy]phosphinyl]cholinhydroxid-inneres Salz,
O-[Hydroxy-[2-(stigmast-5-en-3β-yloxy)äthoxy]phosphinyl]cholinhydroxid-inneres Salz,
Cholest-5-en-3β-yl-2-[[[2-(dimethylamino)äthoxy]hydroxyphosphinyl]oxy]äthylcarbonat,
O-[Hydroxy-[2-[1-(stigmast-5-en-3β-yloxy)formamido]äthoxy]phosphinyl]cholinhydroxid-inneres Salz,
O-[[(RS)-2-Acetoxy-3-[[(cholest-5-en-3β-yloxy)carbonyl]oxy]propoxy)hydroxyphosphinyl]cholinhydroxid-inneres Salz,

O-[[2-[Cholest-5-en-3β-yloxy)carbonyl]-2-methylpropoxy]hydroxyphosphinyl]-cholinhydroxid-inneres Salz und

O-[[(RS)-2-[1-(Cholest-5-en-3β-yloxy)formamido]-3-hydroxypropoxy]hydroxyphosphinyl]cholinhydroxid-inneres Salz.

11. Alkohole der Formel

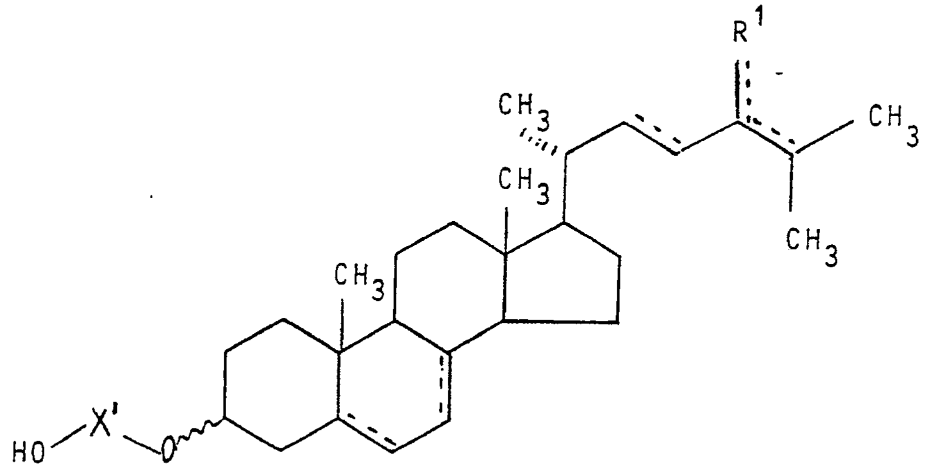

II'

worin

X' eine Gruppe der Formel $-(CH_2)_p-C(Q,Q')-Z'-$, $-(CH_2CH(Y)CH_2-Z'-$, $-CH_2CH(CH_2Y)-Z'$ oder $-(CH_2CH_2O)_q-CH_2CH_2-Z'-$,

Z' eine Gruppe der Formel -OC(O)-, $-OC(O)CH_2-$, $-OCH_2C(O0)-$ oder -N(T)C(O)- ist und

$R^1$, Q, Q', p, q, Y und T die weiter oben angegebene Bedeutung haben.

12. Verbindungen nach Anspruch 1 zur Verwendung als pharmazeutische Wirkstoffe.

13. Verfahren zur Herstellung der Verbindungen nach Anspruch 1, dadurch gekennzeichnet, dass man

a) einen Alkohol der Formel

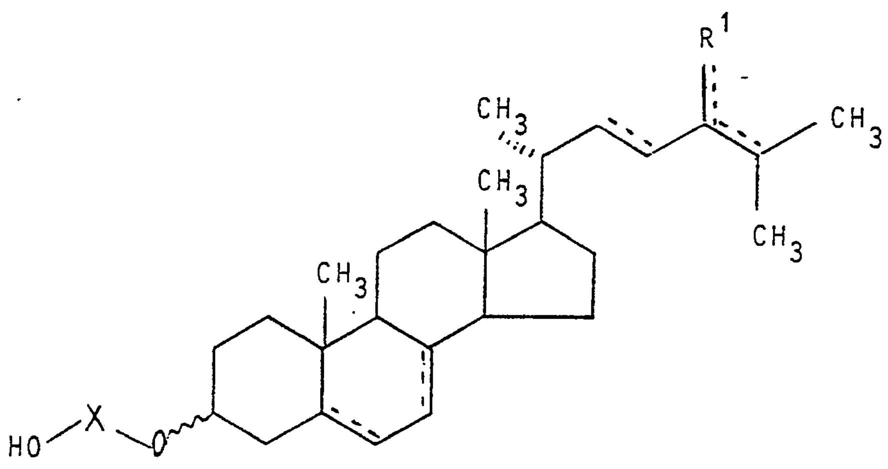

II

unter intermediärem Schutz eines in der Gruppe X vorhandenen Hydroxyrestes Y, mit einem die Gruppe der Formel

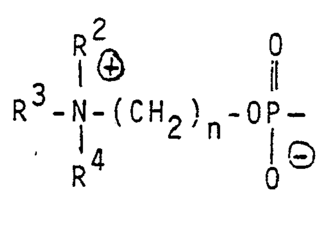

(G)

einführenden Mittel behandelt, und

b) gewünschtenfalls ein ungesättigtes Steroid der Formel I zum gesättigten Steroid hydriert,

c) gewünschtenfalls einen in der Gruppe X eines Steroids der Formel I enthaltenen reaktionsfähigen Rest funktionell abwandelt,

d) gewünschtenfalls ein Steroid der Formel I in ein Salz überführt.

14. Pharmazeutische Präparate auf der Basis einer Verbindung nach Anspruch 1.

15. Verwendung einer Verbindung nach Anspruch 1 bei der Herstellung von die intestinale Resorption von Cholesterin hemmenden und das Plasmacholesterin senkenden Medikamenten.

Patentansprüche für folgende Vertragsstaaten: ES, GR

1. Verfahren zur Herstellung von Steroiden der Formel

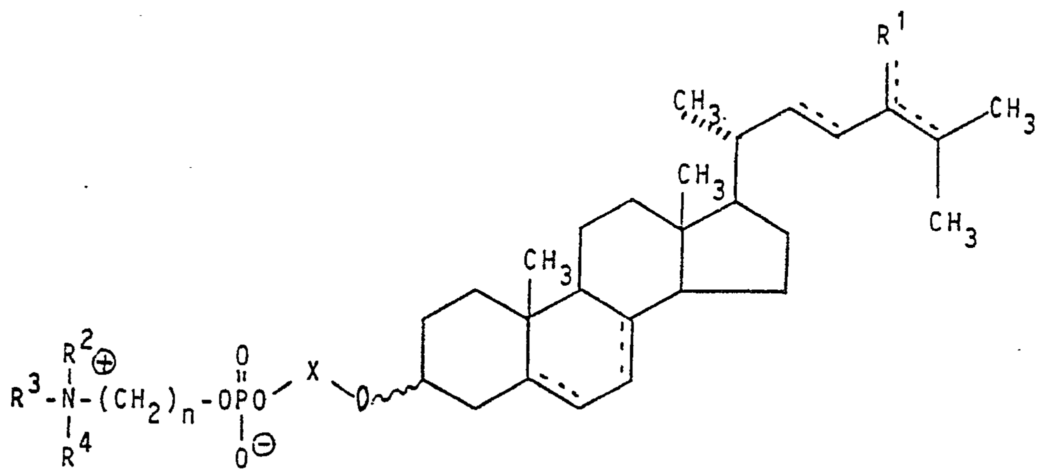

I

worin

$R^1$ Wasserstoff, nieder-Alkyl oder nieder-Alkyliden,

$R^2$, $R^3$ und $R^4$ Wasserstoff oder nieder-Alkyl sind oder zusammen mit dem N-Atom einen 5- oder 6-gliedrigen, gegebenenfalls niederalkylierten, aromatischen oder gesättigten heterocyclischen Rest bilden,

n die Zahl 2, 3 oder 4,

X eine Gruppe der Formel

$-(CH_2)_p-C(Q,Q')-(Z)_{1 \text{ oder } 0}-$,

$-CH_2CH(Y)CH_2-(Z)_{1 \text{ oder } 0}-$,

$-CH_2CH(CH_2Y)-(Z)_{1 \text{ oder} 0}-$ oder

$-(CH_2CH_2O)_q-CH_2CH_2-(Z)_{1 \text{ oder} 0}-$,

q die Zahl 1 oder 2,

Z eine eine Gruppe der Formel $-C(O)-$, $-OC(O)-$, $-OC(O)CH_2-$, $-OCH_2C(O)-$ oder $-N(T)C(O)-$,

Q, Q' und T Wasserstoff oder nieder-Alkyl,

p eine ganze Zahl zwischen 1 und 9 ist und, falls Z Carbonyl ist, auch 0 sein kann,

Y Hydroxy, nieder-Alkoxy, nieder-Alkanoyloxy, Carbamoyloxy oder Mono- oder Di-niederalkyl-carbamoyloxy ist, die gestrichelten C-C-Bindungen in 5(6)-, 7(8)-, 22(23)-, 24(25)- und 24(28)-Stellung fakultativ sind, wobei die Seitenkette entweder gesättigt oder einfach ungesättigt ist,

und physiologisch verträgliche Salze davon, dadurch gekennzeichnet, dass man

a) einen Alkohol der Formel

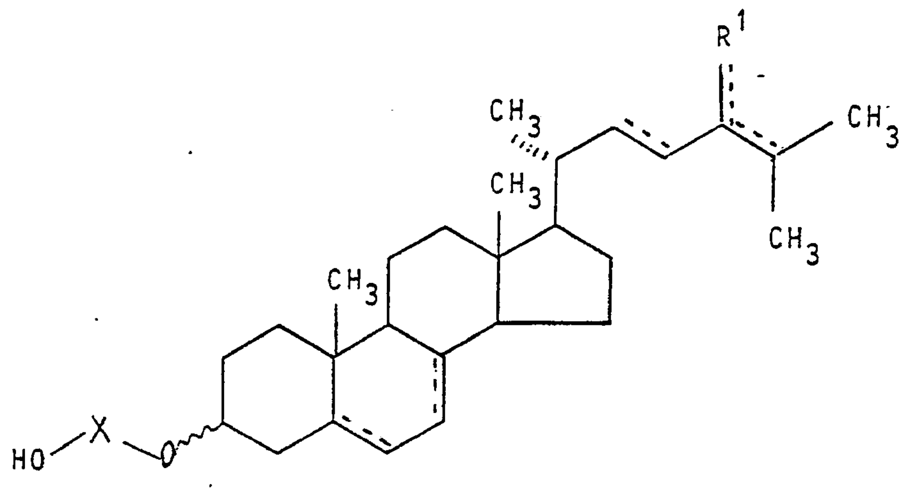

II

unter intermediärem Schutz eines in der Gruppe X vorhandenen Hydroxyrestes Y, mit einem die Gruppe der Formel

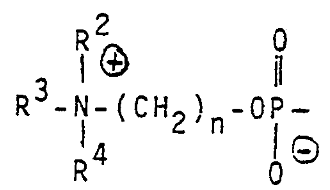

(G)

einführenden Mittel behandelt, und

b) gewünschtenfalls ein ungesättigtes Steroid der Formel I zum gesättigten Steroid hydriert,

c) gewünschtenfalls einen in der Gruppe X eines Steroids der Formel I enthaltenen reaktionsfähigen Rest funktionell abwandelt,

d) gewünschtenfalls ein Steroid der Formel I in ein Salz überführt.

2. Verfahren nach Anspruch 1, worin $R^1$ Wasserstoff oder nieder-Alkyl, insbesondere Aethyl, ist.

3. Verfahren nach Anspruch 1 oder 2, worin $R^2$ Wasserstoff oder nieder-Alkyl und $R^3$ und $R^4$ nieder-Alkyl sind.

4. Verfahren nach Anspruch 1 oder 2, worin $R^2$, $R^3$ und $R^4$ Methyl sind.

5. Verfahren nach Anspruch 1 oder 2, worin $R^2$, $R^3$ und $R^4$ zusammen mit dem N-Atom eine Pyridinium- oder 1-Methylpyrrolidiniumgruppe bilden.

6. Verfahren nach einem der Ansprüche 1-5, worin n die Zahl 2 ist.

7. Verfahren nach einem der Ansprüche 1-6, dadurch gekennzeichnet, dass man Verbindungen mit gesättigtem oder 5(6)-ungesättigtem oder 5(6)- und 22(23)-ungesättigtem Steroidanteil herstellt.

8. Verfahren nach einem der Ansprüche 1-7, worin X die Gruppe $-(CH_2)_2-$, $-(CH_2)_2O(CH_2)_2-$, $-(CH_2)_2O(CH_2)_2O(CH_2)_2-$, $-CH_2CHOHCH_2-$, $-CH_2CH(OCOCH_3)CH_2-$, $-CH_2CH(OCONHCH_3)CH_2N[CH(CH_3)_2]CO-$, $-(CH_2)_2NHCO-$, $-(CH_2)_3NHCO-$, $-CH_2CHOHCH_2OCO-$, $-CH(CH_3)CO-$ oder insbesondere $-(CH_2)_2OCO-$, $-(CH_2)_3OCO-$, $-(CH_2)_4OCO-$, $-(CH_2)_8OCO-$, $-(CH_2)CO-$, $-(CH_2)_2CO-$, $-(CH_2)_3CO-$, oder $-(CH_2)_5CO-$ darstellt.

9. Verfahren nach einem der Ansprüche 1-7, worin X $-CH_2CH(OCOCH_3)CH_2OCO-$, $-CH_2C(CH_3)_2CO-$ oder $-CH_2CH(CH_2OH)NHCO-$ darstellt.

10. Verfahren nach Anspruch 1, dadurch gekennzeichnet, dass man eine Verbindung aus der Gruppe der folgenden herstellt:

O-[ [2-[[(Cholest-5-en-3β-yloxy)carbonyl]oxy]äthoxy]hydroxyphosphinyl]cholinhydroxid-inneres Salz,

O-[Hydroxy-[3-[(3β-stigmastanyloxy)carbonyl]propoxy]phosphinyl]cholinhydroxid-inneres Salz,

O-[[2-(Cholest-5-en-3β-yloxy)äthoxy]hydroxyphosphinyl]cholinhydroxid-inneres Salz und

O-[Hydroxy-[2-[1-(5α-stigmastan-3β-yloxy)formamido]äthoxy]phosphinyl]cholinhydroxid-inneres Salz.

11. Verfahren nach Anspruch 1, dadurch gekennzeichnet, dass man eine Verbindung aus der Gruppe der

folgenden herstellt:
O-[[2-[[(5α-Stigmastan-3β-yloxy)carbonyl]oxy]äthoxy]hydroxyphosphinyl]cholinhydroxid-inneres Salz,
O-[Hydroxy-[2-[[[E)-stigmasta-5,22-dien-3β-yloxy]carbonyl]äthoxy]phosphinyl]cholinhydroxid-inneres Salz,
O-[[3-[[(Cholest-5-en-3β-yloxy)carbonyl]oxy]propoxy]hydroxyphosphinyl]cholinhydroxid-inneres Salz,
O-[[4-[[(Cholest-5-en-3β-yloxy)carbonyl]oxy]butoxy]hydroxyphosphinyl]cholinhydroxid-inneres Salz,
O-[[[8-[[(Cholest-5-en-3β-yloxy)carbonyl]oxy]octyl]oxy]hydroxyphosphinyl]cholinhydroxid-inneres Salz,
O-[[[(Cholest-5-en-3β-yloxy)carbonyl]methoxy]hydroxyphosphinyl]cholinhydroxid-inneres Salz,
O-[[2-[(Cholest-5-en-3β-yloxy)carbonyl]äthoxy]hydroxyphosphinyl]cholinhydroxid-inneres Salz,
O-[Hydroxy-[2-[(3β-stigmastanyloxy)carbonyl]äthoxy]phosphinyl]cholinhydroxid-inneres Salz,
O-[Hydroxy-[2-[(stigmasta-5,22-dien-3β-yloxy)carbonyl]äthoxy]phosphinyl]cholinhydroxid-inneres Salz,
O-[[3-[(Cholest-5-en-3β-yloxy)carbonyl]propoxy]hydroxyphosphinyl]cholinhydroxid-inneres Salz,
O-[Hydroxy-[3-[(E)-stigmasta-5,22-dien-3β-yloxy)carbonyl]propoxy]phosphinyl]cholinhydroxid-inneres Salz,
O-[Hydroxy-[[5-[(5α-stigmastan-3β-yloxy)carbonyl)pentyl]oxy]phosphinyl]cholinhydroxid-inneres Salz,
O-[Hydroxy-[[5-[((E)-stigmasta-5,22-dien-3β-yloxy)carbonyl]pentyl]oxy]phosphinyl]cholinhydroxid-inneres Salz,
O-[Hydroxy-[[[(3β-stigmastanyl)oxy]carbonyl]methoxy]phosphinyl]cholinhydroxid-inneres Salz,
O-[Hydroxy-[[[(E)-stigmasta-5,22-dien-3β-yloxy]carbonyl]methoxy]phosphinyl)cholinhydroxid-inneres Salz,
O-[Hydroxy-[2-(stigmasta-5,22-dien-3β-yloxy)äthoxy]phosphinyl]cholinhydroxid-inneres Salz,
O-[[2-[2-(Cholest-5-en-3β-yloxy)äthoxy]äthoxy]hydroxyphosphinyl]cholinhydroxid-inneres Salz,
O-[Hydroxy-[2-[2-[(E)-stigmasta-5,22-dien-3β-yloxy]äthoxy]äthoxy]phosphinyl]cholinhydroxid-inneres Salz,
O-[[2-[2-[2-(Cholest-5-en-3β-yloxy)äthoxy]äthoxy]äthoxy]hydroxyphosphinyl]cholinhydroxid-inneres Salz,
O-[[2-[(5α-Cholestan-3β-yloxy)carbonyloxy]äthoxy]hydroxyphosphinyl]cholinhydroxid-inneres Salz,
O-[Hydroxy-[2-[2-(3β-stigmastanyloxy)äthoxy]äthoxy]phosphinyl]cholinhydroxid-inneres Salz,
O-[Hydroxy-[2-(3β-stigmastanyloxy)äthoxy]phosphinyl]cholinhydroxid-inneres Salz,
1-[2-[[Hydroxy-[2-[1-(5α-stigmastan-3β-yloxy)formamido]äthoxy]phosphinyl]oxy]äthyl]pyridiniumhydroxid-inneres Salz,
O-[Hydroxy-[2-[1-[(E)-stigmasta-5,22-dien-3β-yloxy]formamido]äthoxy]phosphinyl]cholinhydroxid-inneres Salz,
O-[Hydroxy-[3-[1-[(E)-stigmasta-5,22-dien-3β-yloxy]formamido]propoxy]phosphinyl]cholinhydroxid-inneres Salz,
O-[Hydroxy-[(RS)-3-[N-isopropyl-1-[(E)-stigmasta-5,22-dien-3β-yl]oxyformamido]-2-[(methylcarbamoyl)oxy]-propoxy]phosphinyl]cholinhydroxid-inneres Salz,
O-[[[2-[1-Cholest-5-en-3β-yloxy]formamido]äthoxy)hydroxyphosphinyl]cholinhydroxid-inneres Salz,
O-[[2-[1-(5β-Cholestan-3α-yloxy)formamido]äthoxy]hydroxyphosphinyl]cholinhydroxid-inneres Salz,
1-[2-[[Hydroxy-[3-[1-[(E)-stigmasta-5,22-dien-3β-yloxy]formamido]propoxy]phosphinyl]oxy]äthyl]-pyridiniumhydroxid-inneres Salz,
O-[Hydroxy-[2-[[[(E)-stigmasta-5,22-dien-3β-yloxy]carbonyl]oxy]äthoxy]phosphinyl]cholinhydroxid-inneres Salz,
1-[2-[[[2-[1-[Cholest-5-en-3β-yloxy]formamido]äthoxy]hydroxyphosphinyl]oxy]äthyl]-1-methylpyrrolidiniumhydroxid-inneres Salz,
1-O-(3α,β-Stigmastanyl)-3-O-(RS)-glyceryl-phosphorylcholin,
O-[[(RS)-2-Acetoxy-3-[5a-stigmastan-3α,β-yloxy]propoxy]hydroxyphosphonyl]cholinhydroxid-inneres Salz,
O-[[(RS)-3-[(cholest-5-en-3β-yloxy)carbonyloxy]-2-hydroxypropoxy]hydroxyphosphinyl]cholinhydroxid-inneres Salz,
O-[Hydroxy-(RS)-2-hydroxy-3-[[5α-stigmastan-3β-yloxy)carbonyloxy]propoxy]phosphinyl]cholinhydroxid-inneres Salz und
O-[Hydroxy-[(RS)-2-hydroxy-3-[[(E)-stigmasta-5,22-dien-3β-yloxy]carbonyloxy]propoxy]phosphinyl]-cholinhydroxid-inneres Salz.

12. Verfahren nach Anspruch 1, dadurch gekennzeichnet, dass man eine Verbindung aus der Gruppe der folgenden herstellt:
O-[Hydroxy-[2-[[(stigmast-5-en-3β-yloxy)carbonyl]oxy]äthoxy]phosphinyl]cholinhydroxid-inneres Salz,
O-[Hydroxy-[3-[(stigmast-5-en-3β-yloxy)carbonyl]propoxy]phosphinyl]cholinhydroxid-inneres Salz,
O-[Hydroxy-[2-(stigmast-5-en-3β-yloxy)äthoxy]phosphinyl]cholinhydroxid-inneres Salz,
Cholest-5-en-3β-yl-2-[[[2-(dimethylamino)äthoxy]hydroxyphosphinyl]oxy]äthylcarbonat,
O-[Hydroxy-[2-[1-(stigmast-5-en-3β-yloxy)formamido]äthoxy]phosphinyl]cholinhydroxid-inneres Salz,
O-[[(RS)-2-Acetoxy-3-[[(cholest-5-en-3β-yloxy)carbonyl]oxy]propoxy]hydroxyphosphinyl]cholinhydroxid-inneres Salz,
O-[[2-[Cholest-5-en-3β-yloxy)carbonyl]-2-methylpropoxy]hydroxyphosphinyl]-cholinhydroxid-inneres Salz

und
O-[[(RS)-2-[1-(Cholest-5-en-3β-yloxy)formamido]-3-hydroxypropoxy]hydroxyphosphinyl]cholinhydroxid-inneres Salz.

13. Verfahren zur Herstellung eines pharmazeutischen Präparates auf der Basis eines Steroids der Formel I nach Anspruch 1 oder eines Salzes davon, dadurch gekennzeichnet, dass man eine solche Verbindung zusammen mit einem pharmazeutischen Träger in eine galenische Form bringt.

14. Verwendung einer Verbindung der Formel I nach Anspruch 1 oder eines Salzes davon bei der Herstellung von die intestinale Resorption von Cholesterin hemmenden und das Plasmacholesterin senkenden Medikamenten.

15. Alkohole der Formel

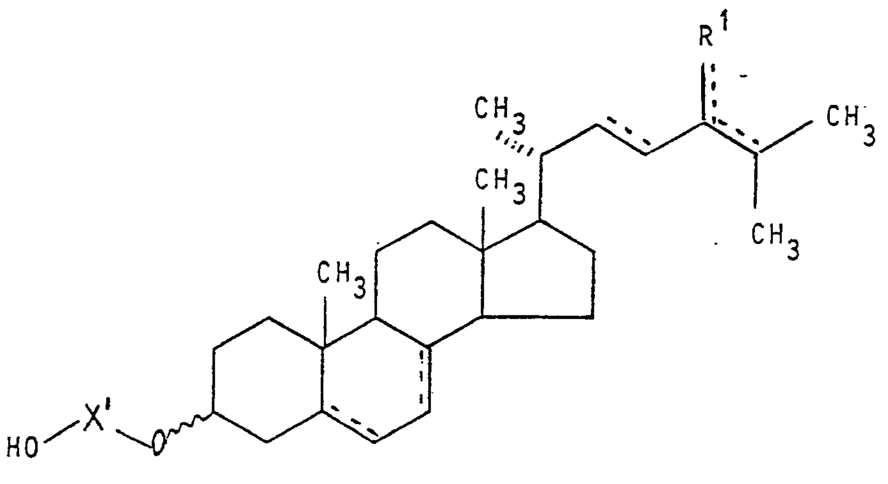

II'

worin
X' eine Gruppe der Formel $-(CH_2)_p-C(Q,Q')-Z'-$, $-CH_2CH(Y)CH_2-Z'-$, $-CH_2CH(CH_2Y)-Z'-$ oder $-(CH_2CH_2O)_q-CH_2CH_2-Z'-$,
Z' eine Gruppe der Formel -OC(O)-, $-OC(O)CH_2-$, $-OCH_2C(O)-$ oder -N(T)C(0)- ist und
$R^1$, Q, Q', p, q, Y und T die weiter oben angegebene Bedeutung haben.